(19) Europäisches Patentamt / European Patent Office / Office européen des brevets

(11) **EP 3 074 774 B1**

(12) **EUROPEAN PATENT SPECIFICATION**

(45) Date of publication and mention of the grant of the patent:
**18.07.2018 Bulletin 2018/29**

(21) Application number: **14802931.7**

(22) Date of filing: **27.11.2014**

(51) Int Cl.:
*G01N 33/92* [(2006.01)]

(86) International application number:
**PCT/EP2014/075874**

(87) International publication number:
**WO 2015/079001 (04.06.2015 Gazette 2015/22)**

(54) **METHODS FOR DETERMINING THE LIPID DISTRIBUTION BETWEEN THE CORE AND THE SHELL OF A LIPOPROTEIN PARTICLE**

VERFAHREN ZUR BESTIMMUNG DER LIPIDVERTEILUNG ZWISCHEN DEM KERN UND DER SCHALE EINES LIPOPROTEINTEILCHENS

PROCÉDÉS PERMETTANT DE DÉTERMINER LA DISTRIBUTION DE LIPIDE ENTRE LE NOYAU ET LA COQUE D'UNE PARTICULE DE LIPOPROTÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2013 EP 13382477**

(43) Date of publication of application:
**05.10.2016 Bulletin 2016/40**

(73) Proprietors:
• **Institut d'Investigació Sanitària Pere Virgili**
  43003 Tarragona (ES)
• **Universitat Rovira I Virgili**
  43003 Tarragona (ES)

(72) Inventors:
• **AMIGÓ GRAU, Núria**
  E-43203 Reus-Tarragona (ES)
• **MALLOL PARERA, Roger**
  E-43204 Reus-Tarragona (ES)
• **CORREIG BLANCHAR, Xavier**
  E-43203 Reus-Tarragona (ES)
• **MASANA MARÍN, Lluís**
  E-43005 Tarragona (ES)
• **RODRÍGUEZ MARTÍNEZ, Miguel Ángel**
  E-43480 Vilaseca,-Tarragona (ES)
• **HERAS IBÁÑEZ, Mercedes**
  E-43202 Reus-Tarragona (ES)
• **PLANA GIL, Núria**
  E-43008 Tarragona (ES)

(74) Representative: **ABG Patentes, S.L.**
**Avenida de Burgos, 16D**
**Edificio Euromor**
**28036 Madrid (ES)**

(56) References cited:
• **ROGER MALLOL ET AL: "Human serum/plasma lipoprotein analysis by NMR: Application to the study of diabetic dyslipidemia", PROGRESS IN NUCLEAR MAGNETIC RESONANCE SPECTROSCOPY, vol. 70, 1 April 2013 (2013-04-01), pages 1-24, XP055108331, ISSN: 0079-6565, DOI: 10.1016/j.pnmrs.2012.09.001**
• **T. TEERLINK: "Combined data from LDL composition and size measurement are compatible with a discoid particle shape", THE JOURNAL OF LIPID RESEARCH, vol. 45, no. 5, 16 February 2004 (2004-02-16), pages 954-966, XP055108545, ISSN: 0022-2275, DOI: 10.1194/jlr.M300521-JLR200**
• **KUMPULA L S ET AL: "Reconsideration of hydrophobic lipid distributions in lipoprotein particles", CHEMISTRY AND PHYSICS OF LIPIDS, LIMERICK, IR, vol. 155, no. 1, 1 September 2008 (2008-09-01), pages 57-62, XP024339169, ISSN: 0009-3084, DOI: 10.1016/J.CHEMPHYSLIP.2008.06.003 [retrieved on 2008-06-20]**

- **L. YETUKURI ET AL: "Composition and lipid spatial distribution of HDL particles in subjects with low and high HDL-cholesterol", THE JOURNAL OF LIPID RESEARCH, vol. 51, no. 8, 1 August 2010 (2010-08-01) , pages 2341-2351, XP055108526, ISSN: 0022-2275, DOI: 10.1194/jlr.M006494**
- **MARCUS STÅHLMAN ET AL: "Dyslipidemia, but not hyperglycemia and insulin resistance, is associated with marked alterations in the HDL lipidome in type 2 diabetic subjects in the DIWA cohort: Impact on small HDL particles", BIOCHIMICA ET BIOPHYSICA ACTA (BBA) - MOLECULAR AND CELL BIOLOGY OF LIPIDS, vol. 1831, no. 11, 27 July 2013 (2013-07-27), pages 1609-1617, XP055108648, ISSN: 1388-1981, DOI: 10.1016/j.bbalip.2013.07.009**

**Description**

**FIELD OF THE INVENTION**

The invention relates to the field of biomedicine, particularly to a method for calculating the distribution of triglycerides and cholesteryl esters between the core and the shell of a HDL lipoprotein particle that transports cholesterol, triglycerides and other lipids in the bloodstream. The invention also relates to methods for monitoring the progression of a subject diagnosed with dyslipidemia, particularly diabetic dyslipidemia, or the effectiveness of a therapy administered to said subject based on this calculation. Methods for identifying compounds suitable for the treatment of dyslipidemia are also provided.

**BACKGROUND OF THE INVENTION**

High levels of cholesterol in blood are known to increase the risk of a variety of diseases, such as atherosclerosis and related disorders such as cardiovascular events like stroke and high blood pressure, and metabolic syndrome, comprising a multitude of phenomena, like diabetes. Recently, it has appeared that the constitution of the blood lipids plays an important role in the development of and/or risk of lipid-related disorders.

Lipids are mainly present in the blood in the form of lipoproteins, which are particles that transport cholesterol, triglycerides and other lipids in the bloodstream. Information on the sizes of the lipoprotein constituents has been assumed as a key issue for a correct diagnosis of lipid-related diseases, but is normally not provided in traditional clinical diagnostics. In the recent years, it has appeared that pathological conditions or the risk thereof can be related to the amount and distribution of lipids over said lipoprotein particles. Thus, lipoprotein particles play a main role in cardiovascular diseases and metabolic disorders.

Lipoproteins are synthesized within the liver and intestines and they carry lipids through the blood stream into peripheral tissues. Lipoproteins are divided into five main classes depending on their size and density: chylomicrons (Q; radii 400-2500 Å), very low density lipoproteins (VLDL; radii 150-400 Å), intermediate density lipoproteins (IDL; radii 125-175 Å), low density lipoproteins (LDL; radii 90-140 Å) and high density lipoproteins (HDL; radii 25-60 Å). Furthermore, these main classes can be divided into different subclasses to obtain a more detailed lipoprotein profile.

It is generally accepted that the structure of a lipoprotein particle is substantially spherical and comprises an inner core and outer shell, wherein non-polar lipids (triglycerides and cholesteryl esters) are found within the core, while polar lipids (phospholipids and free cholesterol) are distributed through a surface monolayer (shell). The protein components of lipoproteins (called apolipoproteins or apoproteins) are located on the surface (shell) together with the polar lipids.

The International Diabetes Federation announced that 366 million people suffered from diabetes in 2011. Type 2 diabetes mellitus (T2DM) accounts for at least 90% of all the cases and it is increasing every year due to the new emergent lifestyles. T2DM, as well as obesity, insulin resistance and metabolic syndrome, are generally associated to a common feature, the atherogenic dyslipidemia, which comprises a triad of increased blood concentrations of small, cholesterol depleted low density lipoproteins (LDL), decreased high density lipoprotein cholesterol (HDL-c) and increased triglycerides. Atherogenic dyslipidemia has emerged as an important risk factor for cardiovascular disease (CVD). LDL-c lowering therapy has been repeatedly demonstrated to reduce CVD risk. However, although the strong inverse association of plasma levels of HDL-c with coronary heart disease found in epidemiological studies, recent events have raised serious questions about the utility of the amount of HDL-c as a good predictor of the cardiovascular risk, and a good target for new therapies. Recently, there exists some controversy about the use of the amount of HDL-c to predict cardiovascular events. Current studies show that the simple fact of raising HDL-c (the "good" cholesterol) does not translate necessarily into a clinical benefit for patients, as when it happens physiologically. In fact, two clinical trials involving therapeutic elevation of HDL-c, based on the administration of niacin and fenofibrate (two drugs that elevate HDL-c levels in blood), were prematurely terminated on the basis of futility [Rader, D.J. & Tall, A.R. Nature Medicine 18, 1344-1346 (2012); Heinecke, J.W. Nature Medicine 18, 1346-1347 (2012)]. Some studies suggest that specific HDL subfractions, and the balance between large and small particles, are the crucial issue to evaluate the cardiovascular risk; and other study remarks the total number of HDL particles as a more suitable and independent risk factor. These discrepancies suggest that finding the appropriate evaluation of the residual risk of these patients is absolutely necessary.

Stählman M. et al (Stählman M. et al. 2013. Biochimica et Biophysica Acta. Molecular and Cell Biology of Lipids, 1831(11):1609-1617) discloses the effects of dyslipidemia on the composition of HDL lipoprotein particles.

Lipoproteins have been analyzed by different techniques including density gradient ultracentrifugation, gradient gel electrophoresis, dynamic light scattering, high performance liquid chromatography, ion mobility analysis and also nuclear magnetic resonance (Mallol R. et al. 2013. Progress in Nuclear Magnetic Resonance Spectroscopy, 70:1-24).

Several models for calculating the spatial distribution of lipids across the core and the shell of a lipoprotein have been developed (Teerlink T. 2004. The Journal of Lipid Research, 45(5):954-966; Kumpula L.S. et al. 2008.

Chemistry and Physics of Lipids, 155(1):57-62; Yetukuri L. et al. 2010. The Journal of Lipid Research, 51(8):2341-2351) although they are not used for routine analysis.

It is therefore necessary to found new biomarkers suitable for predicting the response to a therapy for the treatment of a dyslipidemia, particularly for the treatment of diabetic dyslipidemia. In addition, it would also be necessary to provide a reliable method for analyzing said biomarkers.

## SUMMARY OF THE INVENTION

It has been surprisingly found that core lipoprotein particle lipids (triglycerides and cholesteryl esters) may also be found on the surface (shell) of the lipoprotein particle and that the percentage of triglycerides and cholesteryl esters in the surface (shell) and in the core of the lipoprotein particle is different between subjects suffering a dyslipidemia and healthy subjects. Thus, abnormalities of the spatial lipid distribution may explain the dysfunctional behavior of subjects suffering from a dyslipidemia.

A new approach to characterize in depth the HDL particles fraction of a group of healthy controls and a group of T2DM patients before and after two different interventions with niacin and fenofibrates has been followed. Using NMR studies combined by a biochemical volumetric model the size, the balance of large, medium and small particles and their spatial lipid distribution, was obtained thus giving insights into their functionality. The results demonstrate how these treatments do not reverse the pathological condition although they raise the HDL-cholesterol level and diminish the triglycerides level. These findings were contrasted with an independent analysis of the whole serum obtaining consistent results.

Thus, inventors have developed a method for calculating the distribution of triglycerides and cholesteryl esters between the core and the shell of a HDL lipoprotein based on the comparison between the shell (external) and core (internal) volume ratios calculated by two different methods. Briefly, NMR experiments were used to measure the mean HDL particle radius and the balance between large, medium and small HDL subclasses of healthy controls and T2DM patients. The T2DM patients group was analyzed before and after two widely used pharmacological interventions with niacin and fenofibrates. The obtained radii combined with the classical biochemical information were used together to construct a new volumetric model to analyze the spatial lipid distribution inside the lipoproteins.

The results obtained by the inventors show that HDL particles from a group of subjects suffering from T2DM are smaller than the HDL particles of a healthy group and that the lipids in the inner core were necessarily in a more reduced space than in the inner core of the healthy group, thus suggesting that part of the lipids traditionally considered as being inside the core have to be, necessarily, in the surface of the lipoprotein particle due to volumetric reasons, in strong contrast with the classical model, in which lipoprotein particles are described as spheres with a closed hydrophobic core composed by triglycerides and cholesterol. Applying the method developed by the inventors it has been found that the percentage of the external volume (shell) occupied by the core lipids is higher for the T2DM group than for the healthy group. The inventors also show that the treatments with niacin or fenofibrate, which did not reverse the pathological state, do not reverse substantially said pathological distribution.

This method, which allows calculation of the distribution of triglycerides and cholesteryl esters between the core and the shell of a HDL lipoprotein particle, helps to better evaluate the risk of cardiovascular events and to monitor the progression of a subject diagnosed with dyslipidemia and/or treated with a therapy. Said method may also be useful in the identification of compounds effective for the treatment of a dyslipidemia or capable of producing a dyslipidemia.

Thus, in an aspect, the invention relates to an *in vitro* method for determining the distribution of triglycerides and cholesteryl esters between the core and the shell of a HDL lipoprotein particle from a biofluid sample, said method comprising:

a) calculating the ratio between the shell volume *(Vext)* and the core volume *(Vint)* of a HDL lipoprotein particle by equation [1]:

$$\frac{Vext}{Vint}\,dif = \frac{\frac{4}{3}\pi \cdot R^3 - \frac{4}{3}\pi \cdot (R-x)^3}{\frac{4}{3}\pi \cdot (R-x)^3} = \frac{R^3}{(R-x)^3} - 1$$

$$[1]$$

wherein

R is the hydrodynamic radius of a HDL lipoprotein particle in Å, and
x is the thickness of the HDL lipoprotein shell in Å;

b) calculating the ratio between the shell volume *(Vext)* and the core volume *(Vint)* of a HDL lipoprotein particle by equation [2]:

$$\frac{Vext}{V\operatorname{int}}bio = \frac{A + \alpha \cdot B + \gamma \cdot C}{B \cdot (1-\alpha) + C \cdot (1-\gamma)}$$

[2]

wherein:

A = [FC] $v_{FC}$ + [PL] $v_{PL}$ + [Prot] $v_{Prot}$ per volume unit of the biofluid sample;
B = [TG] $v_{TG}$ per volume unit of the biofluid sample; and
C = [CE] $v_{CE}$ per volume unit of the biofluid sample,

wherein

[FC], [PL], [Prot], [TG] and [CE] are, respectively, the total concentration in mol/ml of free cholesterol, phospholipids, apolipoproteins, triglycerides and cholesteryl esters in a HDL lipoprotein particle; and
$v_{FC}$, $v_{PL}$, $v_{Prot}$, $v_{TG}$ and $v_{CE}$ are, respectively, the molar specific volume in ml/mol of free cholesterol, phospholipids, apolipoproteins, triglycerides and cholesteryl esters in a HDL lipoprotein particle;

and wherein the values of R, x, [FC], [PL], [Prot], [TG], [CE], $v_{FC}$, $v_{PL}$, $v_{Prot}$, $v_{TG}$ and $v_{CE}$ are known or are experimentally determined;
c) assigning a value comprised between 0 and 1 to $\alpha$ and, independently, assigning a value comprised between 0 and 1 to $\gamma$, so that the absolute value of the difference between the ratios calculated in steps a) and b) is equal to or lower than 5% of the ratio calculated in step a); and
d) determining the distribution of triglycerides and cholesteryl esters between the core and the shell of a HDL lipoprotein particle using the values assigned to $\alpha$ and $\gamma$ by calculating parameters $\alpha \cdot B$, $B \cdot (1-\alpha)$, $\gamma \cdot C$ and $C \cdot (1-\gamma)$ wherein

A is the shell volume of a HDL lipoprotein particle occupied by the sum of the volumes of free cholesterol, phospholipids and apolipoproteins per volume unit of the biofluid sample;
$\alpha \cdot B$ is the shell volume of a HDL lipoprotein particle occupied by the volume of triglycerides per volume unit of the biofluid sample;
$\gamma \cdot C$ is the shell volume of a HDL lipoprotein particle occupied by the volume of cholesteryl esters per volume unit of the biofluid sample;
$B \cdot (1-\alpha)$ is the core volume of a HDL lipoprotein particle occupied by the volume of triglycerides per volume unit of the biofluid sample; and $C \cdot (1-\gamma)$ is the core volume of a HDL lipoprotein particle occupied by the volume of cholesteryl esters per volume unit of the biofluid sample.

In another aspect, the invention relates to an *in vitro* method for monitoring the progression of a subject diagnosed with dyslipidemia comprising determining the distribution of triglycerides and cholesteryl esters between the core and the shell of a HDL lipoprotein particle from a biofluid sample, by a method according to the invention and comparing the result with a previous measurement
wherein a reduction of the shell volume of a HDL lipoprotein particle occupied by triglycerides and/or cholesteryl esters with respect to the same values previously measured is indicative that the subject has a good prognosis, or
wherein an increase of the shell volume of a HDL lipoprotein particle occupied by triglycerides and/or cholesteryl esters with respect to the same values previously measured is indicative that the subject has a bad prognosis.
In another aspect, the invention relates to an *in vitro* method for monitoring the effectiveness of a therapy administered to a subject diagnosed with dyslipidemia or for designing a customized therapy for said subject comprising determining the distribution of triglycerides and cholesteryl esters between the core and the shell of a HDL lipoprotein particle from a biofluid sample of said subject before and after said therapy, by a method according to the invention
wherein a reduction of the shell volume of a HDL lipoprotein particle occupied by triglycerides and/or cholesteryl esters after the therapy administered with respect to the same values before the therapy is indicative that the therapy administered is effective, or wherein an increase or no change in the shell volume of a HDL lipoprotein particle occupied by triglycerides and/or cholesteryl esters after the therapy administered with respect to the same values before the therapy is indicative that the therapy administered is ineffective and/or that the subject is in need of an alternative therapy.

In another aspect, the invention relates to an *in vitro* method for the identification of compounds suitable for the treatment of dyslipidemia comprising determining in a biofluid sample of a subject suffering from dyslipidemia and having been treated with a candidate compound, the distribution of triglycerides and cholesteryl esters between the core and the shell of a HDL lipoprotein particle by a method according to the invention wherein a reduction of the shell volume of a HDL lipoprotein particle occupied by triglycerides and/or cholesteryl esters after the treatment with a candidate compound with respect to the same values before the treatment with said candidate compound is indicative that the compound is suitable for the treatment of dyslipidemia, or

wherein an increase or no change in the shell volume of a HDL lipoprotein particle occupied by triglycerides and/or cholesteryl esters after the treatment with a candidate compound with respect to the same values before the treatment with said candidate compound is indicative that the compound is not suitable for the treatment of dyslipidemia.

In another aspect, the invention relates to an *in vitro* method for the identification of compounds capable of producing dyslipidemia comprising determining in a biofluid sample of a subject which has been treated with a candidate compound, the distribution of triglycerides and cholesteryl esters between the core and the shell of a HDL lipoprotein particle by a method according to the invention

wherein an increase in the shell volume of a HDL lipoprotein particle occupied by triglycerides and/or cholesteryl esters after the treatment with a candidate compound with respect to the same values before the treatment with said candidate compound is indicative that the compound is capable of producing dyslipidemia.

In another aspect, the invention relates to a computer program comprising computer program code, which, when executed by a computer device, causes the computer device to carry out the method steps of the method of the invention.

It is also disclosed a computer-implemented method for determining the distribution of triglycerides and cholesteryl esters between the core and the shell of a lipoprotein particle from a biofluid sample according to the method of the invention.

It is also disclosed a computer-implemented method for monitoring the progression of a subject diagnosed with dyslipidemia according to the method of the invention or for monitoring the effectiveness of a therapy administered to a subject diagnosed with dyslipidemia or for designing a customized therapy for said subject according to the method of the invention or for the identification of compounds suitable for the treatment of dyslipidemia according to the method of the invention or for the identification of compounds capable of producing dyslipidemia according to the method of the invention.

## BRIEF DESCRIPTION OF THE DRAWINGS

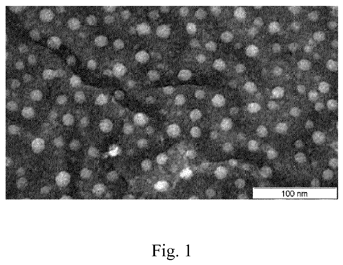

Figure 1 is a photograph of HDL particles obtained by using transmission electronic microscopy (TEM).

## DETAILED DESCRIPTION OF THE INVENTION

The present invention provides methods for calculating the distribution of triglycerides and cholesteryl esters between the core and the shell of a HDL lipoprotein particle and uses thereof.

Method for determining triglycerides and cholesteryl esters distribution between the core and the shell of a HDL lipoprotein particle

Thus, in an aspect, the invention relates to an *in vitro* method for determining, particularly calculating, the distribution of triglycerides and cholesteryl esters between the core and the shell of a HDL lipoprotein particle from a biofluid sample, hereinafter referred to as "first method of the invention", said method comprising:

a) calculating the ratio between the shell volume (*Vext*) and the core volume (*Vint*) of a HDL lipoprotein particle by equation [1]:

$$\frac{Vext}{Vint}\, dif = \frac{\frac{4}{3}\pi \cdot R^3 - \frac{4}{3}\pi \cdot (R-x)^3}{\frac{4}{3}\pi \cdot (R-x)^3} = \frac{R^3}{(R-x)^3} - 1$$

$$[1]$$

wherein

R is the hydrodynamic radius of a HDL lipoprotein particle in Å, and
x is the thickness of the HDL lipoprotein shell in Å;

b) calculating the ratio between the shell volume (*Vext*) and the core volume (*Vint*) of a HDL lipoprotein particle by equation [2]:

$$\frac{Vext}{V\text{int}}bio = \frac{A + \alpha \cdot B + \gamma \cdot C}{B \cdot (1 - \alpha) + C \cdot (1 - \gamma)}$$

[2]

wherein:

A = [FC] $v_{FC}$ + [PL] $v_{PL}$ + [Prot] $v_{Prot}$ per volume unit of the biofluid sample;
B = [TG] $v_{TG}$ per volume unit of the biofluid sample; and
C = [CE] $v_{CE}$ per volume unit of the biofluid sample,

wherein

[FC], [PL], [Prot], [TG] and [CE] are, respectively, the total concentration in mol/ml of free cholesterol, phospholipids, apolipoproteins, triglycerides and cholesteryl esters in a HDL lipoprotein particle; and
$v_{FC}$, $v_{PL}$, $v_{Prot}$, $v_{TG}$ and $v_{CE}$ are, respectively, the molar specific volume in ml/mol of free cholesterol, phospholipids, apolipoproteins, triglycerides and cholesteryl esters in a HDL lipoprotein particle;

and wherein the values of R, x, [FC], [PL], [Prot], [TG], [CE], $v_{FC}$, $v_{PL}$, $v_{Prot}$, $v_{TG}$ and $v_{CE}$ are known or are experimentally determined;

c) assigning a value comprised between 0 and 1 to $\alpha$ and, independently, assigning a value comprised between 0 and 1 to $\gamma$, so that the absolute value of the difference between the ratios calculated in steps a) and b) is equal to or lower than 5% of the ratio calculated in step a); and

d) determining the distribution of triglycerides and cholesteryl esters between the core and the shell of a HDL lipoprotein particle using the values assigned to $\alpha$ and $\gamma$ by calculating parameters $\alpha \cdot B$, B·(1-$\alpha$), $\gamma \cdot C$ and C·(1-$\gamma$) wherein

A is the shell volume of a HDL lipoprotein particle occupied by the sum of the volumes of free cholesterol, phospholipids and apolipoproteins per volume unit of the biofluid sample;
$\alpha \cdot B$ is the shell volume of a HDL lipoprotein particle occupied by the volume of triglycerides per volume unit of the biofluid sample;
$\gamma \cdot C$ is the shell volume of a HDL lipoprotein particle occupied by the volume of cholesteryl esters per volume unit of the biofluid sample;
B·(1-$\alpha$) is the core volume of a HDL lipoprotein particle occupied by the volume of triglycerides per volume unit of the biofluid sample; and
C·(1-$\gamma$) is the core volume of a HDL lipoprotein particle occupied by the volume of cholesteryl esters per volume unit of the biofluid sample.

According to the first method of the invention, a biofluid sample is *in vitro* analyzed for determining, preferably calculating, the distribution of triglycerides and cholesteryl esters between the core and the shell of a HDL lipoprotein particle.

The term "biofluid sample", as used herein, refers to any biofluid sample containing HDL lipoprotein particles. Preferably, the biofluid sample is selected from blood plasma, serum, cerebrospinal fluid, amniotic fluid and a fraction thereof; more preferably is selected from blood plasma, serum and a fraction thereof.

In a particular embodiment, said biofluid sample derives from a subject, preferably a human subject. As used herein, the term "subject" includes a member of a mammalian species, including but not limited to, domestic animals, primates and humans; preferably, the subject is a male or female human being of any age or race. Thus, in a preferred embodiment, the biofluid sample comprises human blood plasma, serum or a fraction thereof.

In a particular embodiment, the first method of the invention is performed over a HDL lipoprotein particle fraction previously isolated from the biofluid sample. In another particular embodiment, the first method of the invention is performed over a whole biofluid sample.

The term "lipoprotein particle", as used herein, refers to negatively charged compositions that comprise a core of non-polar hydrophobic cholesteryl esters and triglycerides surrounded by a surface layer or shell of amphipathic phospholipids with which free cholesterol and apolipoproteins are associated. In the context of the present invention, the shell also contains cholesteryl esters and/or triglycerides. Lipoprotein particles may be characterized by their size and density: chylomicrons (Q; radii 400-2500 Å), very low density lipoproteins (VLDL; radii 150-400 Å), intermediate density lipoproteins (IDL; radii 125-175 Å), low density lipoproteins (LDL; radii 90-140 Å) and high density lipoproteins (HDL; radii 25-60 Å), as well as by the relative amounts of lipid and protein. Furthermore, those main classes can be divided into different subclasses to obtain a more detailed lipoprotein profile. Lipoproteins may also be characterized by the presence or absence of particular modifications (e.g. oxidization, acetylation, enzymatic glycosylation, non-enzymatic glycosylation, etc.).

The method disclosed herein can be performed over any class and subclass of lipoprotein particles. Thus in a disclosure, the lipoprotein particle fraction under study comprises chylomicrons, VLDL, IDL, LDL and/or HDL classes. In another disclosure, the lipoprotein particle fraction under study is selected from the group consisting of chylomicrons, VLDL, IDL, LDL, HDL particles and any combination thereof. The lipoprotein particle fraction under study may contain any subclasss of chylomicrons, VLDL, IDL, LDL or HDL particle classes. In the context of the present invention, the lipoprotein particle or lipoprotein particle fraction is HDL.

According to step a) of the first method of the invention, the ratio between the shell volume (*Vext*) and the core volume (*Vint*) of a HDL lipoprotein particle is calculated by equation [1]. The hydrodynamic radius "R" of a HDL lipoprotein particle may be calculated by any suitable method or equation well-known for the skilled person in the art; nevertheless, in a particular embodiment, the hydrodynamic radius "R" of a HDL lipoprotein particle is calculated according to the Stokes-Einstein equation [3]:

$$ R = \frac{K_B \cdot T}{6\pi\eta \cdot D} $$

$$ [3] $$

wherein

$K_B$ is the Boltzmann constant in $J \cdot K^1$,
T is the absolute temperature in K,
$\eta$ is the viscosity of the solution in Pa·s, and
D is the diffusion coefficient of said HDL lipoprotein particle in $cm^2 \cdot s^{-1}$.

The diffusion coefficient "D" can be obtained by any suitable method, for example, by subjecting the biofluid sample under study to a technique such as bidimensional (2D) diffusion-edited $^1$H-NMR spectroscopy, photon correlation spectroscopy and the like.

Bidimensional (2D) diffusion-edited $^1$H-NMR spectroscopy and photon correlation spectroscopy (also known as "quasi-elastic light scattering") are techniques well-known by the person skilled in the art. In a preferred embodiment the technique used is bidimensional (2D) diffusion-edited $^1$H-NMR spectroscopy.

Alternative methods for calculating the radius "R" of a lipoprotein particle are microscopic techniques such as, without limitation, transmission electronic microscopy and atomic force microscopy.

The thickness of the HDL lipoprotein shell "x" is comprised between 19 and 21 Å, typically about 20 Å [Kumpula L.S. et al., Chemistry and Physics of Lipids, 155:57-62 (2008)].

According to step b) of the first method of the invention, the ratio between the shell volume (*Vext*) and the core volume (*Vint*) of a HDL lipoprotein particle is calculated by equation [2]. This step b) comprises determining the total concentration of free cholesterol, phospholipids, apolipoproteins, triglycerides and cholesteryl esters in a HDL lipoprotein particle. Practically, any method capable of obtaining the total concentration of said molecules in a HDL lipoprotein particle from a biofluid sample can be used.

Cholesterol is an amphipatic lipid. A cholesteryl ester is an ester of cholesterol wherein the ester bond is formed between the carboxylate group of a fatty acid and the hydroxyl group of cholesterol. Illustrative, non-limitative, examples of cholesteryl esters present in a lipoprotein particle are cholesteryl palmitate, cholesteryl stearate, cholesteryl oleate, cholesteryl linoleate and cholesteryl araquidonate [Skipski, V.P. In: Blood Lipids and Lipoproteins. Quantitation, Composition and Metabolism. pp. 471-483 (ed. G.J. Nelson, Wiley-Interscience, New York) (1972)]. Cholesteryl esters have a lower solubility in water than cholesterol and are more hydrophobic. Numerous methods are available for determination of cholesterol concentration, e.g., gravimetric, nephelometric, turbidimetric, or photometric methods, among others. Commercially available kits for quantitative colorimetric/fluorimetric cholesterol and cholesteryl esters determination may be used. Usually, the concentrations of total and free cholesterol (esterified cholesterol being previously precipitated by, for example, digitonin) are determined, whereas the concentration of cholesteryl esters (esterified cholesterol) is calculated from the difference between these two concentrations. Enzymatic determination of cholesterol concentration is specific and sensitive. The principle of reaction is as follows: Cholesterol esterase catalyzes hydrolysis of cholesteryl esters to free cholesterol and free fatty acids. In the presence of cholesterol oxidase, cholesterol is oxidized to $\delta$-4-cholestanetriol to form hydrogen peroxide. In the presence of peroxidase, phenol and 4-aminoantipyrine with hydrogen peroxide yield a red-colored product, quinonimine. The staining intensity is directly proportional to the sample concentration of total cholesterol.

Phospholipids are a class of lipids that are present in the shell of lipoprotein particles and that are a major component of all cell membranes as they can form lipid bilayers. Most phospholipids contain a diglyceride, a phosphate group, and a simple organic molecule such as choline. The structure of the phospholipid molecule generally consists of hydrophobic tails and a hydrophilic head. Illustrative, non-limitative, examples of phospholipids present in a lipoprotein particle are phosphatidylcholine, sphingophospholipids such as sphingomyelin, phosphatidylethanolamine, phosphatidylinositol and phosphatidylserine. Illustrative, non-limitative, methods for determination of phospholipids concentration, include commercially available assay kits for a quantitative colorimetric/fluorimetric phospholipid determination. The principle of reaction is as follows: phospholipids (such as lecithin, lysolecithin and sphingomyelin) are enzymatically hydrolyzed to choline which is determined using choline oxidase and a $H_2O_2$ especific dye. The optical density of the pink colored product at 570nm or fluorescence intensity (530/585 nm) is directly proportional to the phospholipid concentration in the sample.

A triglyceride (or triacylglycerol) is an ester derived from glycerol and three fatty acids which can be found in a lipoprotein particle. Illustrative, non-limitative, fatty acids that can be found in lipoprotein triglycerides are palmitic acid, estearic acid, oleic acid, linoleic acid and araquidonic acid. Triglycerides are blood lipids that help enable the bidirectional transference of adipose fat and blood glucose from the liver. Illustrative, non-limitative, methods for determination of triglycerides concentration, include enzymatic determination. Enzymatic determination of triglycerides is also possible because it is specific and sensitive. The principle of reaction is as follows: Triglycerides are hydrolyzed by a lipase in glycerol and free fatty acids. In the presence of glycerol kinase, glycerol is phosphorylated to glycerol-3-phosphate which is then oxidized with a glycerol phosphate oxidase with formation of hydrogen peroxide. In the presence of peroxidase, 4-chlorophenol and 4-aminoantipyrine with hydrogen peroxide yield a red-colored product, quinonimine. The staining intensity is directly proportional to the sample concentration of triglycerides.

As used herein, the term "apolipoproteins" or "apoproteins", refer to proteins that bind lipids to form lipoproteins and transport lipids through the circulatory system. Apolipoproteins are amphipathic molecules that can surround lipids creating the lipoprotein particle that is itself water-soluble and can thus be carried through water-based circulation (i.e., blood). There are six classes of apolipoproteins (A-H) and several subclasses; in particular, Apo A-I (or Apo A1) is the major protein component of HDL particles wherein Apo A-II (or Apo A2) is also present in a minor concentration. Illustrative, non-limitative, methods for determination of apolipoproteins concentration, include without limitation, colorimetric methods, Western blot or ELISA. If apolipoprotein concentration is determined in an isolated fraction of lipoproteins any method well-known in the art for determining protein concentrations may be used such as enzymatic methods, colorimetric methods (Biuret, Lowry, Bradford, etc.) or immunochemical techniques (Western blot, ELISA, etc.). If apolipoprotein concentration is determined in a biofluid sample, such as plasma or serum sample immunochemical techniques are used in order to obtain a specific detection of apolipoproteins. Illustrative, non-limitative, immunochemical techniques for apolipoprotein detection include immunoturbidimetric techniques, immunonefelometric techniques, radial immunodiffusion, ELISA, electroimmunoanalysis, and radio immunoanalysis.

Once the concentration of free cholesterol, phospholipids and apolipoproteins is determined, parameter "A", i.e., the shell volume of a HDL lipoprotein particle occupied by the sum of the volumes of free cholesterol, phospholipids and apolipoproteins per volume unit of the biofluid sample, is calculated by means of equation [4]:

$$A = [FC]\, v_{FC} + [PL]\, v_{PL} + [Prot]\, v_{Prot} \text{ per volume unit of the biofluid sample} \qquad [4]$$

wherein

[FC], [PL] and [Prot] are, respectively, the total concentration in mol/ml of free cholesterol, phospholipids and apolipoproteins in a HDL lipoprotein particle; and

$v_{FC}$, $v_{PL}$ and $v_{Prot}$ are, respectively, the molar specific volume in ml/mol of free cholesterol, phospholipids and apolipoproteins in a HDL lipoprotein particle.

Further, once the concentration of triglycerides is determined, parameter "B" is calculated by means of equation [5]:

$$B = [TG] \, v_{TG} \text{ per volume unit of the biofluid sample} \qquad [5]$$

wherein

[TG] is the total concentration in mol/ml of triglycerides in a HDL lipoprotein particle; and
$v_{TG}$ is the molar specific volume in ml/mol of triglycerides in a HDL lipoprotein particle.

Once parameter "B" is determined, it can be used for determining the parameter "$\alpha \cdot B$", i.e., the shell volume of a HDL lipoprotein particle occupied by the volume of triglycerides per volume unit of the biofluid sample, as well as parameter "$B \cdot (1-\alpha)$", i.e., the core volume of a HDL lipoprotein particle occupied by the volume of triglycerides per volume unit of the biofluid sample.

Additionally, once the concentration of cholesteryl esters is determined, parameter "C" is calculated by means of equation [6]:

$$C = [CE] \, v_{CE} \text{ per volume unit of the biofluid sample} \qquad [6]$$

wherein

[CE] is the total concentration in mol/ml of cholesteryl esters in a HDL lipoprotein particle; and
$v_{CE}$ is the molar specific volume in ml/mol of cholesteryl esters in a HDL lipoprotein particle.

Once parameter "C" is determined, it can be used for determining the parameter "$\gamma \cdot C$", i.e., the shell volume of a HDL lipoprotein particle occupied by the volume of cholesteryl esters per volume unit of the biofluid sample, as well as parameter "$C \cdot (1-\gamma)$", i.e., the core volume of a HDL lipoprotein particle occupied by the volume of cholesteryl esters per volume unit of the biofluid sample.

The term "molar specific volume" refers to the volume occupied by one mol of a molecule in ml/mol. Methods for calculating the molar specific volume of a substance are well known for the skilled person in the art.

In the first method of the invention the values of R, x, [FC], [PL], [Prot], [TG], [CE], $v_{FC}$, $v_{PL}$, $v_{Prot}$, $v_{TG}$ and $v_{CE}$ are known or are experimentally determined. Said parameters are known when the person carrying out the first method of the invention knows the value of said parameters either because they are known from bibliographical references or because they have been experimentally determined previously by a third person and subsequently provided to the person carrying out the method of the invention. The values of said parameters can also be experimentally determined by the person carrying out the method of the invention by any of the experimental methods disclosed above.

In a particular embodiment, the concentration of free cholesterol, phospholipids, apolipoproteins, triglycerides and cholesteryl esters in a HDL lipoprotein particle is determined by a biochemical method or by a spectroscopic method, for example by NMR, such as $^1$H-NMR spectroscopy. Suitable biochemical methods for determining the concentration of free cholesterol, phospholipids, apolipoproteins, triglycerides and cholesteryl esters have been disclosed previously. Preferably, these biochemical methods are adapted to be used in an automated autoanalyzer such as Cobas.

According to step c) of the first method of the invention, a value comprised between 0 and 1 is assigned to $\alpha$ and, independently, a value comprised between 0 and 1 is assigned to $\gamma$, so that the absolute value of the difference between the ratios calculated in steps a) and b) is equal to or lower than 5% of the ratio calculated in step a), i.e.

$$\frac{\left[\frac{Vext}{Vint} dif - \frac{Vext}{Vint} bio\right]}{\frac{Vext}{Vint} dif} \leq 0.05$$

$\alpha$ and $\gamma$ values are assigned in order to obtain an absolute value of the difference between the ratios calculated in steps a) and b) equal to or lower than 5% of the ratio calculated in step a); preferably lower than 4%; more preferably lower than 3%, even more preferably lower than 2%; yet more preferably lower than 1%; yet preferably lower than 0.5%. In another preferred embodiment $\alpha$ and $\gamma$ values are assigned in order to obtain the same ratio between the shell volume (Vext) and the core volume (Vint) of a HDL lipoprotein particle in steps a) and b). In this step $\alpha$ and $\gamma$ values are assigned and the values that minimize the difference between the ratios calculated in steps a) and b) are selected.

Subsequently, the results obtained in steps a), b) and c) are correlated with the lipid distribution between the core and the shell of a HDL lipoprotein particle. Said correlation involves calculating parameters "$\alpha$·B", "B·(1-$\alpha$)", "$\gamma$·C" and "C·(1-$\gamma$)" as disclosed previously. Briefly, the values assigned to $\alpha$ and $\gamma$ are used to determine the distribution of triglycerides and cholesteryl esters between the core and the shell of a HDL lipoprotein particle by calculating parameters $\alpha$·B, B·(1-$\alpha$), $\gamma$·C and C·(1-$\gamma$) wherein A is the shell volume of a HDL lipoprotein particle occupied by the sum of the volumes of free cholesterol, phospholipids and apolipoproteins per volume unit of the biofluid sample; $\alpha$·B is the shell volume of a HDL lipoprotein particle occupied by the volume of triglycerides per volume unit of the biofluid sample; $\gamma$·C is the shell volume of a HDL lipoprotein particle occupied by the volume of cholesteryl esters per volume unit of the biofluid sample; B·(1-$\alpha$) is the core volume of a HDL lipoprotein particle occupied by the volume of triglycerides per volume unit of the biofluid sample; and C·(1-$\gamma$) is the core volume of a HDL lipoprotein particle occupied by the volume of cholesteryl esters per volume unit of the biofluid sample.

In a particular embodiment, the method according to the invention comprises a previous step wherein the biofluid sample is subjected to bidimensional (2D) diffusion-edited $^1$H-NMR spectroscopy to experimentally determining the hydrodynamic radius "R" of a HDL lipoprotein particle.

In another particular embodiment, the method according to the invention comprises a previous step wherein the biofluid sample is subjected to biochemical methods or spectroscopic methods for determining the concentration of free cholesterol, phospholipids, apolipoproteins, triglycerides and cholesteryl esters. In a preferred embodiment the spectroscopic methods are $^1$H-NMR spectroscopy, preferably bidimensional (2D) diffusion-edited $^1$H-NMR spectroscopy.

In general, a result wherein the mean radius of the lipoprotein particles is lower than the mean radius of the expected lipoprotein particles according to the classical model, i.e., wherein the lipoprotein particles are described as spheres with a closed hydrophobic core composed by triglycerides and cholesterol. A reduction in the size of the lipoprotein particles involves some consequences in the lipid distribution of said lipoprotein particles. Thus, considering lipoproteins as spheres having a 20 Å thick shell, there are spatial restrictions for the molecules to be inside or outside the lipoprotein particles; in fact, in those lipoprotein particles having a smaller size, the lipids in the inner core will be necessarily in a more reduced space, being impossible to occupy only the fixed core volume because the volume where molecules are located is extremely sensitive to small changes in the radii implying huge differences between the volumetric ratio of the external volume and the internal one. Consequently, in order to solve these spatial incoherencies, it has been assumed that part of the lipids that traditionally were considered to be only inside the core (i.e., cholesteryl esters and triglycerides) have to necessarily be in the surface (shell) of the lipoprotein particles for volumetric reasons. This altered distribution could be the responsible of some dysfunctional behavior due to hydrophobic and hydrophilic interactions with the apolipoproteins present in the lipoprotein particles, and probably may have effects on their appropriate conformation.

Example 1 confirms the above hypothesis. Effectively, as it is shown in Example 1 [Table 1a]:

- the mean radius of the lipoprotein particles in the T2DM group was lower than the mean radius of the lipoprotein particles in the control group;
- the % of medium HDL particles subclass in the control group was higher than in the T2DM group; and
- the small HDL particles subclass in the control group was lower than in the T2DM group.

These results show that the HDL particles of the control group are clearly different from the HDL particles of the T2DM group, basically due to the differences of the HDL particle size, and their implications.

As mentioned above, the reduction in the size of the lipoprotein particles involves some consequences in the lipid distribution of HDL particles. This abnormal distribution and the high concentration of triglycerides present in HDL particles from T2DM patients could be the responsible of the HDL particles dysfunctional behavior, due to the hydrophobic and hydrophilic interactions with the apolipoproteins present in the HDL particles, and probably may have effects on their appropriate conformation. In fact, the results shown in Example 1 support the hypothesis that the functionality of HDL particles can be compromised in a pathological state such as T2DM.

In a particular embodiment, the first method of the invention comprises calculating the percentage of triglycerides and cholesteryl esters located in the shell of the HDL lipoprotein particle.

In another particular embodiment, the first method of the invention comprises calculating the percentage of triglycerides and cholesteryl esters located in the core of the HDL lipoprotein particle.

Method for monitoring the progression of a subject diagnosed with dyslipidemia

In another aspect, the invention relates to an *in vitro* method for monitoring the progression of a subject diagnosed with dyslipidemia, hereinafter referred to as "second method of the invention", which comprises determining, particularly calculating, the distribution of triglycerides and cholesteryl esters between the core and the shell of a HDL lipoprotein particle from a biofluid sample, by the first method of the invention and comparing the result with a previous measurement.

The particulars of the first method of the invention have been previously mentioned and are also applicable to the second method of the invention. In the second method of the invention the lipoprotein particle is a HDL particle. In a preferred embodiment the biofluid sample is selected from blood plasma, serum and a fraction thereof.

As used herein, the term "dyslipidemia" refers to an abnormal amount of lipids (e.g. cholesterol and/or fat) in the blood. In developed countries, most dyslipidemias are hyperlipidemias; that is, an elevation of lipids in the blood. This is often due to diet and lifestyle. Prolonged elevation of insulin levels can also lead to dyslipidemia. Likewise, increased levels of O-GlcNAc transferase (OGT) may cause dyslipidemia.

Hyperlipidemia (or hyperlipoproteinemia) involves abnormally elevated levels of any or all lipids and/or lipoproteins in the blood. It is the most common form of dyslipidemia (which includes any abnormal lipid levels). Lipids (fat-soluble molecules) are transported in a protein particle. The size of that particle, or lipoprotein particle, determines its density. The lipoprotein density and type of apolipoproteins it contains determines the fate of the particle and its influence on metabolism. Hyperlipidemias are divided in primary and secondary subtypes. Primary hyperlipidemia is usually due to genetic causes (such as a mutation in a receptor protein), while secondary hyperlipidemia arises due to other underlying causes such as diabetes. Lipid and lipoprotein abnormalities are common in the general population, and are regarded as a modifiable risk factor for cardiovascular disease due to their influence on atherosclerosis. In addition, some forms may predispose to acute pancreatitis.

Hyperlipidemias may basically be classified as either familial (also called primary) caused by specific genetic abnormalities, or acquired (also called secondary) when resulting from another underlying disorder that leads to alterations in plasma lipid and lipoprotein metabolism. Also, hyperlipidemia may be idiopathic, that is, without known cause. Hyperlipidemias are also classified according to which types of lipids are elevated, that is hypercholesterolemia, hypertriglyceridemia or both in combined hyperlipidemia. Elevated levels of lipoprotein may also be classified as a form of hyperlipidemia. Briefly:

Hyperlipoproteinemia type I: Type I hyperlipoproteinemia exists in several forms: (i) Lipoprotein lipase deficiency (Type Ia), due to a deficiency of lipoprotein lipase (LPL) or altered apolipoprotein C2, resulting in elevated chylomicrons; (ii) Familial apoprotein CII deficiency (Type Ib), a condition caused by a lack of lipoprotein lipase activator; and (iii) Chylomicronemia due to circulating inhibitor of lipoprotein lipase (Type Ic). Type I hyperlipoproteinemia usually presents in childhood with eruptive xanthomata and abdominal colic. Complications include retinal vein occlusion, acute pancreatitis, steatosis and organomegaly, and lipaemia retinalis.

Hyperlipoproteinemia type II, by far the most common form, is further classified into type IIa and type IIb, depending mainly on whether there is elevation in the triglyceride level in addition to LDL cholesterol.

Type IIa (familial hypercholesterolemia): This may be sporadic (due to dietary factors), polygenic, or truly familial as a result of a mutation either in the LDL receptor gene on chromosome 19 (0.2% of the population) or the ApoB gene (0.2%). The familial form is characterized by tendon xanthoma, xanthelasma and premature cardiovascular disease. The incidence of this disease is about 1 in 500 for heterozygotes, and 1 in 1,000,000 for homozygotes. HLPIIa is a rare genetic disorder characterized by increased levels of LDL cholesterol in the blood due to the lack of uptake (no Apo B receptors) of LDL particles. This pathology however is the second most common disorder of the various hyperlipoproteinemias, with individuals with a heterozygotic predisposition of 1 in every 500 and individuals with homozygotic predisposition of 1 in every million. These individuals may present with a very unique set of physical characteristics such as: Xanthelasma's (yellow deposits of fat underneath the skin often presenting in the nasal portion of the eye), tendon and tuberous xanthomas, *Arcus juvenilis*, Arterial bruits, claudication, and of course atherosclerosis. Laboratory findings for these individuals are obvious and yet interesting in the fact that their serum cholesterol is 2-3 times greater than normal as well as increased LDL cholesterol but their triglycerides and VLDL values fall in the normal ranges.

Type IIb: The high VLDL levels are due to overproduction of substrates, including triglycerides, acetyl CoA, and an increase in B-100 synthesis. They may also be caused by the decreased clearance of LDL. Prevalence in the population is 10%. Included within this class are (i) Familial combined hyperlipoproteinemia (FCH), (ii) Lysosomal acid lipase deficiency, often called (Cholesteryl ester storage disease), and (iii) Secondary combined hyperlipoproteinemia (usually in the context of metabolic syndrome, for which it is a diagnostic criterion).

Hyperlipoproteinemia type III: This form is due to high chylomicrons and IDL intermediate density lipoprotein). Also known as broad beta disease or dysbetalipoproteinemia, the most common cause for this form is the presence of ApoE E2/E2 genotype. It is due to cholesterol-rich VLDL (β-VLDL). Its prevalence has been estimated to be approximately 1 in 10,000.

Hyperlipoproteinemia type IV: Familial hypertriglyceridemia is an autosomal dominant condition occurring in approximately 1% of the population.

Hyperlipoproteinemia type V: Hyperlipoproteinemia type V is very similar to type I, but with high VLDL in addition to chylomicrons. It is also associated with glucose intolerance and hyperuricemia.

Unclassified familial forms: Non-classified forms are extremely rare: hyperalphalipoproteinemia and polygenic hypercholesterolemia.

Acquired (secondary), also called secondary dyslipoproteinemias, often mimic primary forms of hyperlipidemia and can have similar consequences. They may result in increased risk of premature atherosclerosis or, when associated with marked hypertriglyceridemia, may lead to pancreatitis and other complications of the chylomicronemia syndrome. The most common causes of acquired hyperlipidemia are diabetes mellitus, use of drugs such as diuretics, beta blockers, and estrogens. Other conditions leading to acquired hyperlipidemia include: hypothyroidism, renal failure, nephrotic syndrome, alcohol usage, or some rare endocrine disorders and metabolic disorders. Another acquired cause of hyperlipidemia, although not always included in this category, is postprandial hyperlipidemia, a normal increase following ingestion of food.

In a particular embodiment, the dyslipidemia is selected from hypocholesterolemia, hypercholesterolemia, hypertriglyceridemia, hyperchylomicronemia, dyslipidemia associated with drug therapy, diabetic dyslipidemia and hyperlipoproteinemia types I, IIa, IIb, III, IV and V. In a more particular embodiment, the dyslipidemia is diabetic dyslipidemia. In another particular embodiment the dyslipidemia is selected from hypercholesterolemia, hypertriglyceridemia, hyperchylomicronemia and hyperlipoproteinemia types I, IIa, IIb, III, IV and V.

According to the second method of the invention it is possible to monitor the progression of a subject diagnosed with dyslipidemia by calculating the lipoprotein lipid distribution, namely the distribution of triglycerides and cholesteryl esters, between the core and the shell of a HDL lipoprotein particle from a biofluid sample according to the first method of the invention and comparing the result with a previous measurement. Said previous measurement corresponds to a prior analysis of the lipoprotein lipid distribution, mainly triglycerides and cholesteryl esters, between the core and the shell of a HDL lipoprotein particle from a biofluid sample, performed according to the first method of the invention. The result of the comparison may be indicative of the prognosis, good or bad, of the dyslipidemia. As used herein, the term "prognosis" refers to a medical term for predicting the likely outcome of one's current standing.

In a particular embodiment, the dyslipidemia is diabetic dyslipidemia, namely, type 2 diabetes mellitus (T2DM). According to the second method of the invention, a reduction of the shell volume of a HDL lipoprotein particle occupied by triglycerides and/or cholesteryl esters with respect to the same values previously measured is indicative of a good prognosis, i.e., the disease (dyslipidemia) or the symptoms of the disease are ameliorating. Alternatively, an increase of the shell volume of a HDL lipoprotein particle occupied by triglycerides and/or cholesteryl esters with respect to the same values previously measured is indicative of a bad prognosis, i.e., the disease (dyslipidemia) or the symptoms of the disease are worsening. This is applicable to dyslipidemias and, particularly, to diabetic dyslipidemia.

Method for monitoring the effectiveness of a therapy for dyslipidemia

In another aspect, the invention relates to an *in vitro* method for monitoring the effectiveness of a therapy administered to a subject diagnosed with dyslipidemia or for designing a customized therapy for said subject, hereinafter referred to as "third method of the invention", which comprises determining, particularly calculating, the distribution of triglycerides and cholesteryl esters between the core and the shell of a HDL lipoprotein particle from a biofluid sample from said subject before and after said therapy, by the first method of the invention.

The particulars of the first method of the invention have been previously mentioned and are also applicable to the third method of the invention. In the third method of the invention the lipoprotein particle is a HDL particle. In a preferred embodiment the biofluid sample is selected from blood plasma, serum and a fraction thereof.

The term "dyslipidemia" has been defined previously in connection with the second method of the invention.

As used herein, the term "therapy" or "treatment" collectively refers to the means of any class, hygienic means, pharmacological means, surgical means or physical means, the purpose of which is to prevent and/or cure or relieve a disease or pathology or its symptoms. In an embodiment the therapy is selected from diet therapy, drug therapy, exercise therapy and a combination thereof. In a specific case, said treatment is a pharmacological treatment, i.e., a treatment comprising the administration of a drug to a subject to prevent, relieve and/or cure a disease, e.g., a dyslipidemia, or to relieve, reduce or eliminate one or more symptoms associated with said disease. In the context of the present invention, the treatment applied to the subject comprises diet, exercise and/or is a drug suitable for the treatment of a dyslipidemia, such as a lipid-lowering drug, for example, 3-hydroxy-3-methylglutaryl coenzyme A (HMG-CoA) reductase inhibitors (i.e., the "statins"), the fibrates (e.g., gemfibrozil, clofibrate, and fenofibrate), niacin/nicotinic acid, bile acid binding resins (colestipol and cholestyramine), etc.

Also, as used herein, the term "customized treatment" refers to the design and application of interventions for prevention, diagnosis and treatment adapted to the genetic substrate of the patient and to the molecular profile of the disease (e.g., dyslipidemia).

According to the third method of the invention it is possible to know if a therapy administered to a subject diagnosed with dyslipidemia is effective or not. Thus, in view of the results provided by this invention, the specialist (e.g., a doctor) can optimize the subject therapeutic care by choosing the most suitable drug-based therapy. Therefore, the methods and means provided by the present invention can help the doctors to select the therapy for treating dyslipidemia with a suitable lipid-lowering drug.

According to the third method of the invention, a reduction of the shell volume of a HDL lipoprotein particle occupied by triglycerides and/or cholesteryl esters after the therapy administered with respect to the same value before the therapy, is indicative that the therapy administered is effective, i.e., the disease (dyslipidemia) or the symptoms of disease are ameliorating. Alternatively, an increase or no change of the shell volume of a HDL lipoprotein particle occupied by triglycerides and/or cholesteryl esters after the therapy administered with respect to the same values previously measured before the therapy is indicative that the therapy administered is ineffective, i.e., the disease (dyslipidemia) or the symptoms of disease are worsening and/or that the subject is in need of an alternative therapy. In a preferred embodiment the dyslipidemia is diabetic dyslipidemia.

Method for the identification of compounds suitable for the treatment of dyslipidemia

In another aspect, the invention relates to an *in vitro* method for the identification of compounds suitable for the treatment of dyslipidemia, hereinafter referred to as "fourth method of the invention", which comprises determining in a biofluid sample of a subject suffering from dyslipidemia and having been treated with a candidate compound, the distribution of triglycerides and cholesteryl esters between the core and the shell of a HDL lipoprotein particle by the first method of the invention.

The particulars of the first method of the invention have been previously mentioned and are also applicable to the fourth method of the invention. In the fourth method of the invention the lipoprotein particle is a HDL particle. In a preferred embodiment the biofluid sample is selected from blood plasma, serum and a fraction thereof.

The terms "dyslipidemia" and "treatment" have been defined previously in connection with the second and the third methods of the invention, respectively.

According to the fourth method of the invention it is possible to identify a compound for use in the treatment of dyslipidemia is effective or not. Consequently, in view of the results provided by this invention, the specialist (e.g., a doctor) can optimize the subject therapeutic care by choosing the most suitable drug-based therapy thus excluding those which will be ineffective for the subject to be treated. Therefore, the methods and means provided by the present invention can help the doctors to select the therapy for treating dyslipidemia with a suitable lipid-lowering drug.

According to the fourth method of the invention, a compound is considered as effective for the treatment of dyslipidemia when the shell volume of a HDL lipoprotein particle occupied by triglycerides and/or cholesteryl esters is reduced with respect to the same value in a sample of the same subject before the treatment with said candidate compound.

According to the fourth method of the invention, a compound is considered as suitable (or effective) for the treatment of dyslipidemia when there is a reduction of the shell volume of a HDL lipoprotein particle occupied by triglycerides and/or cholesteryl esters after the treatment with a candidate compound with respect to the same values before the treatment with said candidate compound. Alternatively, a compound is considered not suitable (or ineffective) for the treatment of dyslipidemia when there is an increase or no change in the shell volume of a HDL lipoprotein particle occupied by triglycerides and/or cholesteryl esters after the treatment with a candidate compound with respect to the same values before the treatment with said candidate compound. In a preferred embodiment, the dyslipidemia is diabetic dyslipidemia.

Method for the identification of compounds capable of producing dyslipidemia

In another aspect, the invention relates to an *in vitro* method for the identification of compounds capable of producing dyslipidemia, hereinafter referred to as "fifth method of the invention", which comprises determining in a biofluid sample of a subject which has been treated with a candidate compound, the distribution of triglycerides and cholesteryl esters between the core and the shell of a HDL lipoprotein particle by the first method of the invention.

The particulars of the first method of the invention have been previously mentioned and are also applicable to the fifth method of the invention. In the fifth method of the invention the lipoprotein particle is a HDL particle. In a preferred embodiment the biofluid sample is selected from blood plasma, serum and a fraction thereof.

The term "dyslipidemia" has been defined previously in connection with the second method of the invention.
According to the fifth method of the invention it is possible to identify a compound capable of producing dyslipidemia. In fact, according to the fifth method of the invention, a compound is considered as capable of producing dyslipidemia when the shell volume of a HDL lipoprotein particle occupied by triglycerides and/or cholesteryl esters is increased after the treatment with a candidate compound with respect to the same value in a sample of the same subject before the treatment with said candidate compound. In a preferred embodiment the dyslipidemia is diabetic dyslipidemia.

Computer-implemented methods

In another aspect, the invention relates to a computer program comprising computer program code, which, when executed by a computer device, causes the computer device to carry out the method steps of the first, second, third, fourth or fifth method of the invention.
It is also disclosed a computer-implemented method for determining the distribution of triglycerides and cholesteryl esters between the core and the shell of a lipoprotein particle from a biofluid sample according to the first method of the invention.
It is also disclosed a computer-implemented method for monitoring the progression of a subject diagnosed with dyslipidemia according to the second method of the invention or for monitoring the effectiveness of a therapy administered to a subject diagnosed with dyslipidemia or for designing a customized_therapy for said subject according to the third method of the invention or for the identification of compounds suitable for the treatment of dyslipidemia according to the fourth method of the invention or for the identification of compounds capable of producing dyslipidemia according to the fifth method of the invention.
The present document discloses:

[1].An *in vitro* method for calculating the distribution of triglycerides and cholesteryl esters between the core and the shell of a lipoprotein particle from a biofluid sample, said method comprising:

a) calculating the ratio between the shell volume *(Vext)* and the core volume *(Vint)* of a lipoprotein particle by equation [1]:

$$\frac{Vext}{Vint}\,dif = \frac{\frac{4}{3}\pi \cdot R^3 - \frac{4}{3}\pi \cdot (R-x)^3}{\frac{4}{3}\pi \cdot (R-x)^3} = \frac{R^3}{(R-x)^3} - 1$$

$$[1]$$

wherein

R is the hydrodynamic radius of a lipoprotein particle in Å, and
x is the thickness of the lipoprotein shell in Å;

b) calculating the ratio between the shell volume (*Vext*) and the core volume (*Vint*) of a lipoprotein particle by equation [2]:

$$\frac{Vext}{V\,int}\,bio = \frac{A + \alpha \cdot B + \gamma \cdot C}{B \cdot (1-\alpha) + C \cdot (1-\gamma)}$$

$$[2]$$

wherein

A is the shell volume of a lipoprotein particle occupied by the sum of the volumes of free cholesterol, phospholipids and apolipoproteins per volume unit of the biofluid sample;

α·B is the shell volume of a lipoprotein particle occupied by the volume of triglycerides per volume unit of the biofluid sample;

γ·C is the shell volume of a lipoprotein particle occupied by the volume of cholesteryl esters per volume unit of the biofluid sample;

B·(1-α) is the core volume of a lipoprotein particle occupied by the volume of triglycerides per volume unit of the biofluid sample;

C·(1-γ) is the core volume of a lipoprotein particle occupied by the volume of cholesteryl esters per volume unit of the biofluid sample; and

wherein:

A = [FC] $v_{FC}$ + [PL] $v_{PL}$ + [Prot] $v_{Prot}$ per volume unit of the biofluid sample;
B = [TG] $v_{TG}$ per volume unit of the biofluid sample; and
C = [CE] $v_{CE}$ per volume unit of the biofluid sample,

wherein

[FC], [PL], [Prot], [TG] and [CE] are, respectively, the total concentration in mol/ml of free cholesterol, phospholipids, apolipoproteins, triglycerides and cholesteryl esters in a lipoprotein particle; $v_{FC}$, $v_{PL}$, $v_{Prot}$, $v_{TG}$ and $v_{CE}$ are, respectively, the molar specific volume in ml/mol of free cholesterol, phospholipids, apolipoproteins, triglycerides and cholesteryl esters in a lipoprotein particle;

c) assigning a value comprised between 0 and 1 to α and, independently, assigning a value comprised between 0 and 1 to γ, wherein the absolute value of the difference between the ratios calculated in steps a) and b) is equal to or lower than 5% of the ratio calculated in step a); and

d) correlating the results obtained in steps a), b) and c) with the distribution of triglycerides and cholesteryl esters between the core and the shell of a lipoprotein particle.

[2].The *in vitro* method according to [1], wherein the hydrodynamic radius "R" of a lipoprotein particle is calculated according to the Stokes-Einstein equation [3]:

$$R = \frac{K_B \cdot T}{6\pi\eta \cdot D}$$

[3]

wherein

$K_B$ is the Boltzmann constant in J·K$^1$,
T is the absolute temperature in K,
$\eta$ is the viscosity of the solution in Pa·s, and
D is the diffusion coefficient of said lipoprotein particle in cm$^2$·s$^{-1}$.

[3].The *in vitro* method according to [1] or [2], wherein the thickness of the lipoprotein shell "x" is comprised between 19 and 21 Å.

[4].The *in vitro* method according to any one of [1], [2] or [3], wherein the concentration of free cholesterol, phospholipids, apolipoproteins, triglycerides and cholesteryl esters in a lipoprotein particle is determined by a biochemical method or by a spectroscopic method.

[5].The *in vitro* method according to any one of [1] to [4], further comprising calculating the percentage of triglycerides and cholesteryl esters located in the shell of the lipoprotein particle.

[6].The *in vitro* method according to any one of [1] to [5], further comprising calculating the percentage of triglycerides and cholesteryl esters located in the core of the lipoprotein particle.

[7].The *in vitro* method according to any one of [1] to [6], wherein the biofluid sample is selected from blood plasma, serum and a fraction thereof.

[8].The *in vitro* method according to any one of [1] to [7], wherein said method is performed over a lipoprotein particle fraction previously isolated from the biofluid sample.

[9].The *in vitro* method according to any one of [1] to [8], wherein said method is performed over the whole biofluid sample.

[10]. An *in vitro* method for monitoring the progression of a subject diagnosed with dyslipidemia comprising calculating the distribution of triglycerides and cholesteryl esters between the core and the shell of a lipoprotein particle from a biofluid sample, by a method according to any one of [1] to [9] and comparing the result with a previous measurement.

[11]. An *in vitro* method for monitoring the effectiveness of a therapy administered to a subject diagnosed with dyslipidemia or for designing a customized therapy for said subject comprising calculating the distribution of triglycerides and cholesteryl esters between the core and the shell of a lipoprotein particle from a biofluid sample from said subject before and after said therapy, by a method according to any one of [1] to [9].

[12]. The *in vitro* method for monitoring the effectiveness of a therapy administered to a subject diagnosed with dyslipidemia according to [11], wherein a reduction of the shell volume of a lipoprotein particle occupied by triglycerides and/or cholesteryl esters after the therapy administered with respect to the same value before the therapy, is indicative that the therapy administered is effective.

[13]. The *in vitro* method for designing a customized therapy for a subject diagnosed with dyslipidemia according to [12], wherein an increase or no change in the shell volume of a lipoprotein particle occupied by triglycerides and/or cholesteryl esters after the therapy administered with respect to the same value before the therapy, is indicative that the subject is in need of an alternative therapy.

[14]. An *in vitro* method for the identification of compounds suitable for the treatment of dyslipidemia comprising determining in a biofluid sample of a subject suffering from dyslipidemia and having been treated with a candidate compound, wherein said biofluid sample comprises blood plasma, serum or a fraction thereof, the distribution of triglycerides and cholesteryl esters between the core and the shell of a lipoprotein particle by a method according to any one of [1] to [9].

[15]. The *in vitro* method according to any one of [10] to [14], wherein the dyslipidemia is diabetic dyslipidemia.

The invention is described hereinafter by way of the following examples which are to be construed as merely illustrative and not limitative of the invention.

**EXAMPLE 1**

**Lipoprotein lipid distribution in type 2 diabetes mellitus patients**

**1. Materials & Methods**

**Subjects**

Study subjects: The participants in this study included 26 healthy subjects and 29 patients suffering from type 2 diabetes mellitus (T2DM) with atherogenic dyslipidemia, i.e., triglycerides levels above 200 mg/dL and HDL-cholesterol under 60 mg/dL. T2DM patients underwent two lipid-lowering therapies. Firstly, they were divided into two groups and fenofibrate and niacin were administered, respectively, for 12 weeks. After a washout period of 6 weeks, they interchanged the treatments for 12 weeks more.

**HDL fraction analysis**

Lipoprotein fractionation: Prior to the NMR analysis, HDL particle fractions were obtained by plasma ultracentrifugation [Mallol, R. et al. Analytical and bioanalytical chemistry 402, 2407-2415 (2012)].
NMR feature extraction: $^1$H NMR spectra were recorded on a Bruker Advance III 600 spectrometer operating at 310 K. Double stimulated echo (DSTE) pulse program with bipolar gradient pulses and a longitudinal eddy current delay (LED) was used as previously reported [Mallol et al. (2012) cited *supra*].

<u>Radius extraction</u>: The DSTE methyl signal was fitted with one lorentzian curve to obtain the averaged diffusion coefficient ("D") of the lipoprotein particles. The hydrodynamic radii ("R") of the lipoprotein particle fractions were extracted from the Stokes-Einstein equation [3]:

$$R = \frac{K_B \cdot T}{6\pi\eta \cdot D}$$

$$[3]$$

wherein

R is the hydrodynamic radius of a lipoprotein particle in Å,

$K_B$ is the Boltzmann constant in J·K$^{-1}$,

T is the absolute temperature in K,

$\eta$ is the viscosity of the solution in Pa·s, and

D is the diffusion coefficient of a lipoprotein particle in cm$^2$·s$^{-1}$, wherein said diffusion coefficient is obtained by subjecting the biofluid sample under study to 2D diffusion-edited $^1$H-NMR spectroscopy.

<u>Particle size distribution</u>: The methylene LED signal had enough resolution to be fitted with three lorentzians corresponding to three different HDL particle subclasses (large, medium and small HDL particles). The methylene group of the lipids contained in lipoprotein particles (mainly esterified cholesterol and triglycerides) resonate at slightly shifted frequencies depending on the size of the particle that carries them, larger particles resonating at higher frequencies. The area below each lorentzian curve is proportional to the concentration of particles of this particular subclass. The relative concentration of each subclass provides an estimation of the size distribution of HDL particles.

Finally, some samples were observed using transmission electronic microscopy (TEM), in order to visualise the homogeneity and the dispersion of the HDL particles (Figure 1).

<u>Determination of the concentration of phospholipids, apolipoproteins, triglycerides, cholesteryl esters and free cholesterol in plasma lipoprotein particles</u>: For the HDL fraction analysis the concentration of phospholipids (PL), apolipoproteins (Prot), triglycerides (TG), cholesteryl esters (CE) and free cholesterol (FC) of the HDL particle fractions was measured by biochemical classical methods [Mallol et al. (2012) cited *supra*].

**Compositional reconstruction of HDL subfraction**

A mathematical optimization was used to determine how lipids were distributed inside lipoproteins. The classical model describes lipoproteins as spheres with a superficial shell of 20 Å thickness composed by phospholipids, apolipoproteins and free cholesterol covering a core of esterified cholesterol and triglycerides. However, a modified model considering part of esterified cholesterol and triglycerides to be in the superficial shell was used.

In order to estimate the part of triglycerides and esterified cholesterol present in the shell the following protocol was followed:

- Firstly, the ratio between the volume of the external part (i.e., the 20 Å shell) and the internal part (core) using the measured radii was computed. The geometric expression for a known radius sphere wherein the thickness of the shell of the lipoprotein particle is shown in equation [7]:

$$Ratio_{geometry} = \frac{\frac{4}{3}\pi \cdot R^3 - \frac{4}{3}\pi \cdot (R - 20)^3}{\frac{4}{3}\pi \cdot (R - 20)^3}$$

$$[7]$$

wherein

R is the hydrodynamic radius of a lipoprotein particle in Å,

- Secondly, the ratio between the shell volume (*Vext*) and the core volume (*Vint*) of a lipoprotein particle was calculated by equation [2]:

$$\frac{Vext}{V\,\mathrm{int}}bio = \frac{A + \alpha \cdot B + \gamma \cdot C}{B \cdot (1-\alpha) + C \cdot (1-\gamma)}$$

$$[2]$$

wherein

A is the shell volume of a lipoprotein particle occupied by the sum of the volumes of free cholesterol, phospholipids and apolipoproteins per volume unit of the biofluid sample;

$\alpha \cdot B$ is the shell volume of a lipoprotein particle occupied by the volume of triglycerides per volume unit of the biofluid sample;

$\gamma \cdot C$ is the shell volume of a lipoprotein particle occupied by the volume of cholesteryl esters per volume unit of the biofluid sample;

$B \cdot (1-\alpha)$ is the core volume of a lipoprotein particle occupied by the volume of triglycerides per volume unit of the biofluid sample;

$C \cdot (1-\gamma)$ is the core volume of a lipoprotein particle occupied by the volume of cholesteryl esters per volume unit of the biofluid sample; and

wherein:

A = [FC] $v_{FC}$ + [PL] $v_{PL}$ + [Prot] $v_{Prot}$ per volume unit of the biofluid sample;

B = [TG] $v_{TG}$ per volume unit of the biofluid sample; and

C = [CE] $v_{CE}$ per volume unit of the biofluid sample,

wherein

[FC], [PL], [Prot], [TG] and [CE] are, respectively, the total concentration in mol/ml of free cholesterol, phospholipids, apolipoproteins, triglycerides and cholesteryl esters in a lipoprotein particle;

$v_{FC}$, $v_{PL}$, $v_{Prot}$, $v_{TG}$ and $v_{CE}$ are, respectively, the molar specific volume in ml/mol of free cholesterol, phospholipids, apolipoproteins, triglycerides and cholesteryl esters in a lipoprotein particle.

- Thirdly, the difference between the biochemical volumetric ratio and the geometrical ratio allowing part of the core lipids (triglycerides and cholesteryl esters) to be in the superficial shell of the lipoprotein particle was minimized; in order to do that, a value comprised between 0 and 1 was assigned to $\alpha$ and, independently, a value comprised between 0 and 1 was assigned to $\gamma$, so that absolute value of the difference between the ratios calculated in steps a) and b) is equal to or lower than 5% of the ratio calculated in step a).

Finally, the results obtained are correlated with the lipid distribution between the core and the shell of a lipoprotein particle. The values of parameters "$\alpha \cdot B$", "$B \cdot (1-\alpha)$", "$\gamma \cdot C$" and "$C \cdot (1-\gamma)$" were calculated.

**Statistical analysis**

A statistical Mann-Whitney U test was performed to identify significant differences in the fraction of the traditionally core lipids in the external shell between the healthy group [control] and the T2DM group; followed by a Wilcoxon signed-rank test to evaluate the treatment effects for paired samples.

**2. Results**

**Fraction analysis**

Table 1a reports the main HDL particle parameters obtained from the fraction analysis. The mean radius for the healthy group was higher than for the T2DM group, 4.7 nm and 4.5 nm, respectively (p=0.002).

The healthy group presented a 43% of medium HDL particle subclass, higher than the 31% of the T2DM group (p=0.014). In contrast, the healthy group presented a 42% of the small HDL particles subclass, much less than the 57% of the T2DM group, (p=0.041). The analysis of the averaged sizes confirmed this distribution.

Table 1b shows that any of the treatments changed the particle size distribution and the radius of the T2DM group in a significant way. However, the treatment effects were in opposite directions: Fenofibrate treatment tended to shift the distribution to small particles, though the Niacin treatment presented a tendency to the healthy state, incrementing the relative concentration of medium HDL particles subclass (p=0.015). The treatment effects on the radius were consistent (p=0.042).

**Table 1a**

**HDL particles fraction analysis**
**Comparison of control subjects (healthy group) and basal T2DM patients**

|  | Control (n=26) | T2DM (n=29) | P |
| --- | --- | --- | --- |
| Radius (nm) | 4.7 [4.6-4.8] | 4.5 [4.4-4.6] | 0.002 |
| Subclasses Balance (%) |  |  |  |
|     Large HDL | 15 [9-23] | 14 [8-18] | 0.571 |
|     Medium HDL | 43 [34-51] | 31 [21-44] | 0.014 |
|     Small HDL | 42 [28-58] | 57 [32-64] | 0.041 |
| Relative (%) Volume |  |  |  |
|   External shell |  |  |  |
|     Core Lipids | 3 [0-6] | 6 [4-7] | 0.011 |
|     Apolipoprotein | 53 [51-56] | 54 [51-56] | 0.679 |
|     Phospholipids | 37 [34-39] | 34 [31-37] | 0.022 |
|     Free Cholesterol | 7 [6-8] | 6 [5-7] | 0.010 |
|   Internal Core |  |  |  |
|     Cholesteryl esters | 70 [63-74] | 57 [52-64] | <0.0001 |
|     Triglycerides | 30 [26-37] | 43 [36-48] | <0.0001 |

**Table 1b**
**HDL particles fraction analysis**
**Comparison of T2DM patients before and after the treatments**

|  | DM2 (n=21) | Fenofibrate (n=21) | $p_1$ | Niacin (n=21) | $p_2$ | $p_3$ |
| --- | --- | --- | --- | --- | --- | --- |
| Radius (nm) | 4.5 [4.4-4.6] | 4.4 [4.4-4.7] | 0.305 | 4.6 [4.3-4.7] | 0.259 | 0.042 |
| Subclasses Balance (%) |  |  |  |  |  |  |
|     Large HDL | 14 [8-18] | 15 [6-20] | 0.927 | 16 [9-19] | 0.378 | 0.625 |
|     Medium HDL | 32 [27-47] | 31 [24-37] | 0.212 | 38 [33-40] | 0.136 | 0.015 |
|     Small HDL | 58 [30-64] | 54 [44-63] | 0.330 | 46 [42-55] | 0.114 | 0.059 |
| Relative (%) Volume |  |  |  |  |  |  |
|   External Shell |  |  |  |  |  |  |
|     Core Lipids | 5 [4-7] | 5 [4-7] | 0.958 | 4 [2-8] | 0.689 | 0.881 |
|     Apolipoprotein | 53 [50-56] | 55 [53-59] | 0.205 | 56 [53-58] | 0.170 | 0.958 |
|     Phospholipids | 35 [33-37] | 33 [29-37] | 0.305 | 31 [29-38] | 0.170 | 0.434 |
|     Free Cholesterol | 6 [5-7] | 5 [4-6] | 0.140 | 6.3 [6-7] | 0.434 | 0.159 |
|   Internal Core |  |  |  |  |  |  |
|     Cholesteryl esters | 57 [51-63] | 60 [52-68] | 0.434 | 60 [52-69] | 0.205 | 0.217 |
|     Triglycerides | 43 [37-49] | 40 [32-48] | 0.434 | 40 [31-48] | 0.205 | 0.217 |

**Table 1.** The table shows firstly the mean radius for each group. Secondly, the averaged area of the three lorentzians corresponding to the relative particle concentration of each subclass (large, medium and small). Finally, the percentage (%) of the surface volume occupied by the traditionally core lipids, the apolipoproteins, the phospholipids and the free cholesterol; and the percentage (%) of the inner core volume occupied by cholesteryl esters and triglycerides. All the results are expressed as Median $\pm$ 25-75. **Table 1a.** Comparative between controls and T2DM. **Table 1b.** Treatment effects: Comparative between the basal state and the post fenofibrate state ($p_1$) and the post niacin state ($p_2$); comparative between treatments ($p_3$).

**Whole plasma analysis**

Table 2 reports the HDL particle parameters extracted from the plasma analysis. The percentage of the external (shell) volume occupied by the core lipids and the percentage of the inner (core) volume occupied by the triglycerides depends on the HDL particles subclass. In a decreasing order (large, medium and small subclass) the healthy group presented a 2%, 11% and 16% of the core lipids located in the external shell and a 22%, 19% and 18% of the triglycerides located in the inner core, compared to the 5%, 12% and 17% of the core lipids and a 43%, 38% and 35% of the triglycerides for the T2DM group, (p<0.0001 for all cases).

Table 2b shows that only the niacin treatment tended to the healthy state, diminishing both the percentage of the core lipids located in the external shell and the percentage of triglycerides present in the inner core for all HDL particles subclasses.

**Table 2a**
**HDL particles analysis from whole plasma**
**Comparison of control subjects and basal T2DM patients**

|  | Controls (n=26) | T2DM (n=29) | p |
|---|---|---|---|
| Relative (%) Volume |  |  |  |
| External shell |  |  |  |
| Core Lipids (large HDL) | 2 [0-3] | 5 [3-6] | <0.0001 |
| Core Lipids (medium HDL) | 11 [11-11] | 12 [11-14] | <0.0001 |
| Core Lipids (small HDL) | 16 [16-16] | 17 [16-18] | <0.0001 |
| Inner core |  |  |  |
| Triglycerides (large HDL) | 22 [19-25] | 43 [30-56] | <0.0001 |
| Triglycerides (medium HDL) | 19 [17-23] | 38 [25-50] | <0.0001 |
| Triglycerides (small HDL) | 18 [15-24] | 35 [24-43] | <0.0001 |

**Table 2b**
**HDL particles analysis from whole plasma**
**Comparison of T2DM patients before and after the treatments**

|  | T2DM (n=21) | Fenofibrate (n=21) | $p_1$ | Niacin (n=21) | $p_2$ | $p_3$ |
|---|---|---|---|---|---|---|
| Relative (%) Volume |  |  |  |  |  |  |
| External shell |  |  |  |  |  |  |
| Core Lipids (large HDL) | 5 [4-6] | 6 [4-8] | 0.693 | 4 [3-5] | 0.010 | 0.001 |
| Core Lipids (medium HDL) | 12 [11-14] | 12 [11-13] | 0.378 | 11 [11-12] | 0.005 | 0.006 |
| Core Lipids (small HDL) | 17 [16-18] | 17 [17-18] | 0.224 | 17 [16-17] | 0.004 | 0.006 |
| Inner Core |  |  |  |  |  |  |
| Triglycerides (large HDL) | 49 [34-56] | 53 [37-62] | 0.615 | 34 [25-49] | 0.008 | 0.001 |
| Triglycerides (medium HDL) | 39 [27-53] | 38 [27-42] | 0.429 | 29 [21-38] | 0.004 | 0.004 |
| Triglycerides (small HDL) | 36 [24-43] | 35 [26-39] | 0.429 | 28 [21-35] | 0.005 | 0.004 |

**Table 2.** The table shows firstly the % of the surface volume occupied by the traditionally core lipids for each subclass of HDL (large, medium and small) and the % of the inner core volume occupied by the triglycerides. All the results are expressed as Median $\pm$ 25-75. **Table 2a.** Comparative between controls and T2DM. **Table 2b.** Treatment effects: Comparative between the basal state and the post fenofibrate state ($p_1$) and the post niacin state ($p_2$); comparative

between treatments ($p_3$).

### 3. Discussion

The preceding analysis strongly supports the hypothesis that HDL particles functionality can be compromised in a pathological state such as T2DM. These results indicated that the HDL particles of a healthy group are clearly different from the HDL particles of a T2DM group, basically due to the differences of the HDL particle size, and their implications.

The reduction in size had consequences in the lipid distribution of the HDL particles. Considering lipoproteins as spheres with a shell of a 20 Å thickness there are spatial restrictions for the molecules to be inside or outside the lipoprotein particles. Consequently, for the pathological group, which presented a smaller size, the lipids in the inner core were necessarily in a more reduced space than in the case of the healthy ones, being impossible to occupy only the fixed core volume because the volume where molecules are located is extremely sensitive to small changes in the radii implying huge differences between the volumetric ratio of the external volume and the internal one.

To solve these spatial incoherencies, it has been assumed that part of the traditionally lipids inside the core (cholesteryl esters and triglycerides) does necessarily to be in the lipoprotein surface (shell) of the lipoprotein particles for volumetric reasons.

This abnormal distribution and the high concentration of triglycerides present in T2DM HDL particles, could be the responsible of the HDL particles dysfunctional behavior, due to the hydrophobic and hydrophilic interactions with the apolipoproteins present in the HDL particles, and probably may have effects on their appropriate conformation.

### Claims

1.  An *in vitro* method for determining the distribution of triglycerides and cholesteryl esters between the core and the shell of a HDL lipoprotein particle from a biofluid sample, said method comprising:

    a) calculating the ratio between the shell volume *(Vext)* and the core volume *(Vint)* of a HDL lipoprotein particle by equation [1]:

    $$\frac{Vext}{Vint} \, dif = \frac{\frac{4}{3}\pi \cdot R^3 - \frac{4}{3}\pi \cdot (R-x)^3}{\frac{4}{3}\pi \cdot (R-x)^3} = \frac{R^3}{(R-x)^3} - 1$$

    $$[1]$$

    wherein

    R is the hydrodynamic radius of a HDL lipoprotein particle in Å, and
    x is the thickness of the HDL lipoprotein shell in Å;

    b) calculating the ratio between the shell volume *(Vext)* and the core volume *(Vint)* of a HDL lipoprotein particle by equation [2]:

    $$\frac{Vext}{V\mathrm{int}} \, bio = \frac{A + \alpha \cdot B + \gamma \cdot C}{B \cdot (1-\alpha) + C \cdot (1-\gamma)}$$

    $$[2]$$

    wherein:

    A = [FC] $v_{FC}$ + [PL] $v_{PL}$ + [Prot] $v_{Prot}$ per volume unit of the biofluid sample;

B = [TG] $v_{TG}$ per volume unit of the biofluid sample; and
C = [CE] $v_{CE}$ per volume unit of the biofluid sample,

wherein

[FC], [PL], [Prot], [TG] and [CE] are, respectively, the total concentration in mol/ml of free cholesterol, phospholipids, apolipoproteins, triglycerides and cholesteryl esters in a HDL lipoprotein particle; and
$v_{FC}$, $v_{PL}$, $v_{Prot}$, $v_{TG}$ and $v_{CE}$ are, respectively, the molar specific volume in ml/mol of free cholesterol, phospholipids, apolipoproteins, triglycerides and cholesteryl esters in a HDL lipoprotein particle;

and wherein the values of R, x, [FC], [PL], [Prot], [TG], [CE], $v_{FC}$, $v_{PL}$, $v_{Prot}$, $v_{TG}$ and $v_{CE}$ are known or are experimentally determined;
c) assigning a value comprised between 0 and 1 to $\alpha$ and, independently, assigning a value comprised between 0 and 1 to $\gamma$, so that the absolute value of the difference between the ratios calculated in steps a) and b) is equal to or lower than 5% of the ratio calculated in step a); and
d) determining the distribution of triglycerides and cholesteryl esters between the core and the shell of a HDL lipoprotein particle using the values assigned to $\alpha$ and $\gamma$ by calculating parameters $\alpha \cdot B$, $B \cdot (1-\alpha)$, $\gamma \cdot C$ and $C \cdot (1-\gamma)$ wherein

A is the shell volume of a HDL lipoprotein particle occupied by the sum of the volumes of free cholesterol, phospholipids and apolipoproteins per volume unit of the biofluid sample;
$\alpha \cdot B$ is the shell volume of a HDL lipoprotein particle occupied by the volume of triglycerides per volume unit of the biofluid sample;
$\gamma \cdot C$ is the shell volume of a HDL lipoprotein particle occupied by the volume of cholesteryl esters per volume unit of the biofluid sample;
$B \cdot (1-\alpha)$ is the core volume of a HDL lipoprotein particle occupied by the volume of triglycerides per volume unit of the biofluid sample; and
$C \cdot (1-\gamma)$ is the core volume of a HDL lipoprotein particle occupied by the volume of cholesteryl esters per volume unit of the biofluid sample.

2. The *in vitro* method according to claim 1, wherein the hydrodynamic radius "R" of a HDL lipoprotein particle is calculated according to the Stokes-Einstein equation [3]:

$$R = \frac{K_B \cdot T}{6\pi\eta \cdot D}$$

[3]

wherein

$K_B$ is the Boltzmann constant in J·K[1],
T is the absolute temperature in K,
$\eta$ is the viscosity of the solution in Pa·s, and
D is the diffusion coefficient of said HDL lipoprotein particle in $cm^2 \cdot s^{-1}$.

3. The *in vitro* method according to any of claims 1 or 2 comprising a previous step wherein the biofluid sample is subjected to bidimensional (2D) diffusion-edited $^1$H-NMR spectroscopy to experimentally determining the hydrodynamic radius "R" of a HDL lipoprotein particle.

4. The *in vitro* method according to any one of claims 1 to 3, wherein the concentration of free cholesterol, phospholipids, apolipoproteins, triglycerides and cholesteryl esters in a HDL lipoprotein particle is determined by a biochemical method or by a spectroscopic method.

5. The *in vitro* method according to any one of claims 1 to 4, further comprising calculating the percentage of triglycerides and cholesteryl esters located in the shell of the HDL lipoprotein particle.

6. The *in vitro* method according to any one of claims 1 to 5, further comprising calculating the percentage of triglycerides and cholesteryl esters located in the core of the HDL lipoprotein particle.

7. The *in vitro* method according to any one of claims 1 to 6, wherein the biofluid sample is selected from blood plasma, serum and a fraction thereof.

8. The *in vitro* method according to any one of claims 1 to 7, wherein said method is performed over a HDL lipoprotein particle fraction previously isolated from the biofluid sample.

9. The *in vitro* method according to any one of claims 1 to 8, wherein said method is performed over the whole biofluid sample.

10. An *in vitro* method for monitoring the progression of a subject diagnosed with dyslipidemia comprising determining the distribution of triglycerides and cholesteryl esters between the core and the shell of a HDL lipoprotein particle from a biofluid sample, by a method according to any one of claims 1 to 9 and comparing the result with a previous measurement
    wherein a reduction of the shell volume of a HDL lipoprotein particle occupied by triglycerides and/or cholesteryl esters with respect to the same values previously measured is indicative that the subject has a good prognosis, or wherein an increase of the shell volume of a HDL lipoprotein particle occupied by triglycerides and/or cholesteryl esters with respect to the same values previously measured is indicative that the subject has a bad prognosis.

11. An *in vitro* method for monitoring the effectiveness of a therapy administered to a subject diagnosed with dyslipidemia or for designing a customized therapy for said subject comprising determining the distribution of triglycerides and cholesteryl esters between the core and the shell of a HDL lipoprotein particle from a biofluid sample from said subject before and after said therapy, by a method according to any one of claims 1 to 9
    wherein a reduction of the shell volume of a HDL lipoprotein particle occupied by triglycerides and/or cholesteryl esters after the therapy administered with respect to the same values before the therapy is indicative that the therapy administered is effective, or
    wherein an increase or no change in the shell volume of a HDL lipoprotein particle occupied by triglycerides and/or cholesteryl esters after the therapy administered with respect to the same values before the therapy is indicative that the therapy administered is ineffective and/or that the subject is in need of an alternative therapy.

12. An *in vitro* method for the identification of compounds suitable for the treatment of dyslipidemia comprising determining in a biofluid sample of a subject suffering from dyslipidemia and having been treated with a candidate compound, the distribution of triglycerides and cholesteryl esters between the core and the shell of a HDL lipoprotein particle by a method according to any one of claims 1 to 9
    wherein a reduction of the shell volume of a HDL lipoprotein particle occupied by triglycerides and/or cholesteryl esters after the treatment with a candidate compound with respect to the same values before the treatment with said candidate compound is indicative that the compound is suitable for the treatment of dyslipidemia, or
    wherein an increase or no change in the shell volume of a HDL lipoprotein particle occupied by triglycerides and/or cholesteryl esters after the treatment with a candidate compound with respect to the same values before the treatment with said candidate compound is indicative that the compound is not suitable for the treatment of dyslipidemia.

13. An *in vitro* method for the identification of compounds capable of producing dyslipidemia comprising determining in a biofluid sample of a subject which has been treated with a candidate compound, the distribution of triglycerides and cholesteryl esters between the core and the shell of a HDL lipoprotein particle by a method according to any one of claims 1 to 9
    wherein an increase in the shell volume of a HDL lipoprotein particle occupied by triglycerides and/or cholesteryl esters after the treatment with a candidate compound with respect to the same values before the treatment with said candidate compound is indicative that the compound is capable of producing dyslipidemia.

14. The *in vitro* method according to any one of claims 10 to 13, wherein the dyslipidemia is diabetic dyslipidemia.

15. A computer program comprising computer program code, which, when executed by a computer device, causes the computer device to carry out the method steps of the method according to any of claims 1 to 14.

**Patentansprüche**

1. In-vitro-Verfahren zum Bestimmen der Verteilung von Trigylceriden und Cholesterinestern zwischen dem Kern und der Schale eines HDL-Lipoprotein-Partikels aus einer Bioflüssigkeitsprobe, wobei das Verfahren umfasst:

   a) Berechnen des Verhältnisses zwischen dem Schalenvolumen *(Vext)* und dem Kernvolumen *(Vint)* eines HDL-Lipoprotein-Partikels mittels Gleichung [1]:

$$\frac{Vext}{Vint}\, dif = \frac{\frac{4}{3}\pi \cdot R^3 - \frac{4}{3}\pi \cdot (R-x)^3}{\frac{4}{3}\pi \cdot (R-x)^3} = \frac{R^3}{(R-x)^3} - 1$$

$$[1]$$

   wobei

   R der hydrodynamische Radius eines HDL-Lipoprotein-Partikels in Å ist, und
   x die Dicke der HDL-Lipoprotein-Schale in Å ist;

   b) Berechnen des Verhältnisses zwischen dem Schalenvolumen *(Vext)* und dem Kernvolumen *(Vint)* eines HDL-Lipoprotein-Partikels mittels Gleichung [2]:

$$\frac{Vext}{V\,\mathrm{int}}\, bio = \frac{A + \alpha \cdot B + \gamma \cdot C}{B \cdot (1 - \alpha) + C \cdot (1 - \gamma)}$$

$$[2]$$

   wobei:

   A = [FC] $v_{FC}$ + [PL] $v_{PL}$ + [Prot] $v_{Prot}$ pro Volumeneinheit der Bioflüssigkeitsprobe;
   B = [TG] $v_{TG}$ pro Volumeneinheit der Bioflüssigkeitsprobe; und
   C = [CE] $v_{CE}$ pro Volumeneinheit der Bioflüssigkeitsprobe,

   wobei

   [FC], [PL], [Prot], [TG] und [CE] jeweils die Gesamtkonzentration in mol/ml an freiem Cholesterin, Phospholipiden, Apolipoproteine, Triglyceriden und Cholesterinestern in einem HDL-Lipoprotein-Partikel sind; und
   $v_{FC}$, $v_{PL}$, $v_{Prot}$, $v_{TG}$ und $v_{CE}$ jeweils das molare spezifische Volumen in ml/mol an freiem Cholesterin, Phospholipiden, Apolipoproteinen, Triglyceriden und Cholesterinestern in einem HDL-Lipoprotein-Partikel sind;

   und wobei die Werte R, x, [FC], [PL], [Prot], [TG], [CE], $v_{FC}$, $v_{PL}$, $v_{Prot}$, $v_{TG}$ und $v_{CE}$ bekannt sind oder experimentell bestimmt werden;
   c) Zuweisen eines Wertes, der zwischen 0 und 1 umfasst ist, zu $\alpha$, und, unabhängig, Zuweisen eines Wertes, der zwischen 0 und 1 umfasst ist, zu $\gamma$, sodass der absolute Wert des Unterschieds zwischen den in Schritten a) und b) berechneten Verhältnissen gleich oder niedriger als 5% des in Schritt a) berechneten Verhältnisses ist; und
   d) Bestimmen der Verteilung an Triglyceriden und Cholesterinestern zwischen dem Kern und der Schale eines HDL-Lipoprotein-Partikels unter Verwendung der $\alpha$ und $\gamma$ zugeordneten Werte durch Berechnen der Parameter $\alpha\cdot B$, $B\cdot(1-\alpha)$, $\gamma\cdot C$ und $C\cdot(1-\gamma)$, wobei

   A das Schalenvolumen eines HDL-Lipoprotein-Partikels ist, das von der Summe der Volumina an freiem Cholesterin, Phospolipiden und Apolipoproteinen pro Volumeneinheit der Bioflüssigkeitsprobe eingenommen wird;

α·B das Schalenvolumen eines HDL-Lipoprotein-Partikels ist, das durch das Volumen an Trigylceriden pro Volumeneinheit der Bioflüssigkeitsprobe eingenommen wird;

γ·C das Schalenvolumen eines HDL-Lipoprotein-Partikels ist, das durch das Volumen an Cholesterinestern pro Volumeneinheit der Bioflüssigkeitsprobe eingenommen wird;

B·(1-α) das Kernvolumen eines HDL-Lipoprotein-Partikels ist, das durch das Volumen an Trigylceriden pro Volumeneinheit der Bioflüssigkeitsprobe eingenommen wird;

C·(1-γ) das Kernvolumen eines HDL-Lipoprotein-Partikels ist, das durch das Volumen der Cholesterinestern pro Volumeneinheit der Bioflüssigkeitsprobe eingenommen wird.

2. In-vitro-Verfahren nach Anspruch 1, wobei der hydrodynamische Radius "R" eines HDL-Lipoprotein-Partikels gemäß der Stokes-Einstein-Gleichung [3] berechnet wird:

$$R = \frac{K_B \cdot T}{6\pi\eta \cdot D}$$

[3]

wobei

$K_B$ die Boltzmannkonstante in $J \cdot K^{-1}$ ist,
T die absolute Temperatur in K ist,
η die Viskosität der Lösung in Pa·s ist, und
D der Diffusionskoeffizient des HDL-Lipoprotein-Partikels in $cm^2 \cdot s^{-1}$ ist.

3. In-vitro-Verfahren nach einem der Ansprüche 1 oder 2, umfassend einen vorherigen Schritt, in dem die Bioflüssigkeitsprobe einer zweidimensionalen (2D) Diffusionseditierten $^1$H-NMR-Spektroskopie unterzogen wird, um den hydrodynamischen Radius "R" eines HDL-Lipoprotein-Partikels experimentell zu bestimmen.

4. In-vitro-Verfahren nach einem der Ansprüche 1 - 3, wobei die Konzentration an freiem Cholesterin, Phospholipiden, Apolipoproteinen, Trigylceriden und Cholesterinestern in einem HDL-Lipoprotein-Partikel mittels eines biochemischen Verfahrens oder mittels eines spektroskopischen Verfahrens bestimmt wird.

5. In-vitro-Verfahren nach einem der Ansprüche 1 - 4, weiterhin umfassend, dass Berechnen des prozentualen Anteils an Trigylceriden und Cholesterinestern, die sich in der Schale des HDL-Lipoprotein-Partikels befinden.

6. In-vitro-Verfahren nach einem der Ansprüche 1 - 4, weiterhin umfassend das Berechnen des prozentualen Anteils an Trigylceriden und Cholesterinestern, die sich in dem Kern des HDL-Lipoprotein-Partikels befinden.

7. In-vitro-Verfahren nach einem der Ansprüche 1 - 6, wobei die Bioflüssigkeitsprobe ausgewählt ist aus Blutplasma, Serum und einer Fraktion davon.

8. In-vitro-Verfahren nach einem der Ansprüche 1 - 7, wobei das Verfahren an einer HDL-Lipoprotein-Partikel-Fraktion durchgeführt wird, die zuvor aus der Bioflüssigkeitsprobe isoliert worden ist.

9. In-vitro-Verfahren nach einem der Ansprüche 1 - 8, wobei das Verfahren an der gesamten Bioflüssigkeitsprobe durchgeführt wird.

10. In-vitro-Verfahren zum Überwachen des Fortschritts eines Patienten mit der Diagnose Dyslipidämie, umfassend das Bestimmen der Verteilung von Trigylceriden und Cholesterinestern zwischen dem Kern und der Schale eines HDL-Lipoprotein-Partikels aus einer Bioflüssigkeitsprobe mittels eines Verfahrens nach einem der Ansprüche 1 - 9 und Vergleichen des Ergebnisses mit einer vorherigen Messung, wobei eine Verringerung des Schalenvolumens eines HDL-Lipoprotein-Partikels, das von Trigylceriden und/oder Cholesterinestern eingenommen wird, in Bezug auf dieselben, zuvor gemessenen Werte, darauf hinweist, dass der Patient eine gute Prognose hat, oder,
wobei eine Zunahme des Schalenvolumens eines HDL-Lipoprotein-Partikels, das von Trigylceriden und/oder Cholesterinestern eingenommen wird, in Bezug auf dieselben zuvor gemessenen Werte, darauf hinweist, dass der Patient eine schlechte Prognose hat.

11. In-vitro-Verfahren zum Überwachen der Wirksamkeit einer an einen Patienten mit der Diagnose Dyslipidämie verabreichten Therapie oder zum Entwerfen einer maßgeschneiderten Therapie für den Patienten, umfassend das Bestimmen der Verteilung von Trigylceriden und Cholesterinestern zwischen dem Kern und der Schale eines HDL-Lipoprotein-Partikels aus einer Bioflüssigkeitsprobe von dem Patienten vor und nach der Therapie, mit einem Verfahren nach einem der Ansprüche 1 - 9,
wobei eine Verringerung des Schalenvolumens eines HDL-Lipoprotein-Partikels, das von Trigylceriden und/oder Cholesterinestern nach der verabreichten Therapie eingenommen wird, in Bezug auf dieselben Werte vor der Therapie, darauf hinweist, dass die verabreichte Therapie wirksam ist, oder
wobei eine Zunahme oder keine Veränderung hinsichtlich des Schalenvolumens eines HDL-Lipoprotein-Partikels, das von Trigylceriden und/oder Cholesterinestern nach der verabreichten Therapie eingenommen wird, in Bezug auf dieselben Werte vor der Therapie, darauf hinweist, dass die Therapie unwirksam ist und/oder dass der Patient eine alternative Therapie benötigt.

12. In-vitro-Verfahren zur Identifizierung von Verbindungen, die zur Behandlung von Dyslipidämie geeignet sind, umfassend das Bestimmen in einer Bioflüssigkeitsprobe eines Patienten, der an Dyslipidämie leidet und der mit einer Kandidatenverbindung behandelt worden ist, der Verteilung von Trigylceriden und Cholesterinestern zwischen dem Kern und der Schale eines HDL-Lipoprotein-Partikels mit einem Verfahren nach einem der Ansprüche 1 - 9,
wobei eine Verringerung des Schalenvolumens eines HDL-Lipoprotein-Partikels, das von Trigylceriden und/oder Cholesterinestern nach der Behandlung mit einer Kandidatenverbindung eingenommen wird, in Bezug auf dieselben Werte vor der Behandlung mit der Kandidatenverbindung, darauf hinweist, dass die Verbindung für die Behandlung von Dyslipidämie geeignet ist, oder
wobei eine Zunahme oder keine Veränderung hinsichtlich des Schalenvolumens eines HDL-Lipoprotein-Partikels, das von Trigylceriden und/oder Cholesterinestern nach der Behandlung mit der Kandidatenverbindung eingenommen wird, in Bezug auf dieselben Werte vor der Behandlung mit der Kandidatenverbindung, darauf hinweist, dass die Verbindung für die Behandlung von Dyslipidämie nicht geeignet.

13. In-vitro-Verfahren zur Identifizierung von Verbindungen, die in der Lage sind, Dyslipidämie zu erzeugen, umfassend das Bestimmen in einer Bioflüssigkeitsprobe eines Patienten, der mit einer Kandidatenverbindung behandelt worden ist, der Verteilung von Trigylceriden und Cholesterinestern zwischen dem Kern und der Schale eines HDL-Lipoprotein-Partikels mit einem Verfahren nach einem der Ansprüche 1 - 9,
wobei eine Zunahme hinsichtlich des Schalenvolumens eines HDL-Lipoprotein-Partikels, das von Trigylceriden und/oder Cholesterinestern nach der Behandlung mit einer Kandidatenverbindung eingenommen wird, in Bezug auf dieselben Werte vor der Behandlung mit der Kandidatenverbindung, darauf hinweist, dass die Verbindung in der Lage ist, eine Dyslipidämie zu erzeugen.

14. In-vitro-Verfahren nach einem der Ansprüche 10 - 13, wobei die Dyslipidämie eine Diabetes- Dyslipidämie ist.

15. Computerprogramm, umfassend Computerprogramm-Code, der bei Ausführung durch einen Computer den Computer dazu bringt, die Verfahrensschritte des Verfahrens nach einem der Ansprüche 1 - 14 durchzuführen.

**Revendications**

1. Procédé *in vitro* pour la détermination de la distribution des triglycérides et des esters de cholestéryle entre le noyau et la coque d'une particule de lipoprotéine HDL issue d'un échantillon de liquide biologique, ledit procédé comprenant:

a) le calcul du rapport entre le volume de la coque (*Vext*) et le volume du noyau (*Vint*) d'une particule de lipoprotéine HDL par l'équation [1]:

$$\frac{Vext}{Vint}\, dif = \frac{\frac{4}{3}\pi \cdot R^3 - \frac{4}{3}\pi \cdot (R-x)^3}{\frac{4}{3}\pi \cdot (R-x)^3} = \frac{R^3}{(R-x)^3} - 1$$

$$[1]$$

dans laquelle

R représente le rayon hydrodynamique d'une particule de lipoprotéine HDL en Å, et
x représente l'épaisseur de la coque de la lipoprotéine HDL en Å;

b) le calcul du rapport entre le volume de la coque (*Vext*) et le volume du noyau (*Vint*) d'une particule de lipoprotéine HDL par l'équation [2]:

$$\frac{Vext}{V\,int}\, bio = \frac{A + \alpha \cdot B + \gamma \cdot C}{B \cdot (1-\alpha) + C \cdot (1-\gamma)}$$

$$[2]$$

dans laquelle:

A = [FC] $v_{FC}$ + [PL] $v_{PL}$ + [Prot] $v_{Prot}$ par unité de volume de l'échantillon de liquide biologique;
B = [TG] $v_{TG}$ par unité de volume de l'échantillon de liquide biologique; et
C = [CE] $v_{CE}$ par unité de volume de l'échantillon de liquide biologique,

dans laquelle

[FC], [PL], [Prot], [TG] et [CE] représentent, respectivement, la concentration totale en mol/ml du cholestérol libre, des phospholipides, des apolipoprotéines, des triglycérides et des esters de cholestéryle dans une particule de lipoprotéine HDL; et
$v_{FC}$, $v_{PL}$, $v_{Prot}$, $v_{TG}$ et $v_{CE}$ représentent, respectivement, le volume spécifique molaire en ml/mol du cholestérol libre, des phospholipides, des apolipoprotéines, des triglycérides et des esters de cholestéryle dans une particule de lipoprotéine HDL;

et dans lequel les valeurs de R, x, [FC], [PL], [Prot], [TG], [CE], $v_{FC}$, $v_{PL}$, $v_{Prot}$, $v_{TG}$ et $v_{CE}$ sont connues ou sont déterminées expérimentalement;
c) l'attribution d'une valeur comprise entre 0 et 1 à $\alpha$ et, indépendamment, l'attribution d'une valeur comprise entre 0 et 1 à $\gamma$, de telle façon que la valeur absolue de la différence entre les rapports calculés dans les étapes a) et b) est inférieure ou égale à 5 % du rapport calculé dans l'étape a); et
d) la détermination de la distribution des triglycérides et des esters de cholestéryle entre le noyau et la coque d'une particule de lipoprotéine HDL en utilisant les valeurs attribuées à $\alpha$ et $\gamma$ en calculant les paramètres $\alpha \cdot B$, $B \cdot (1-\alpha)$, $\gamma \cdot C$ et $C \cdot (1-\gamma)$ dans lequel

A représente le volume de la coque d'une particule de lipoprotéine HDL occupé par la somme des volumes du cholestérol libre, des phospholipides et des apolipoprotéines par unité de volume de l'échantillon de liquide biologique;
$\alpha \cdot B$ représente le volume de la coque d'une particule de lipoprotéine HDL occupé par le volume des triglycérides par unité de volume de l'échantillon de liquide biologique;
$\gamma \cdot G$ représente le volume de la coque d'une particule de lipoprotéine HDL occupé par le volume des esters de cholestéryle par unité de volume de l'échantillon de liquide biologique;
$B \cdot (1-\alpha)$ représente le volume du noyau d'une particule de lipoprotéine HDL occupé par le volume des triglycérides par unité de volume de l'échantillon de liquide biologique; et

C·(1-γ) représente le volume du noyau d'une particule de lipoprotéine HDL occupé par le volume des esters de cholestéryle par unité de volume de l'échantillon de liquide biologique.

**2.** Procédé *in vitro* selon la revendication 1, dans lequel le rayon hydrodynamique « R » d'une particule de lipoprotéine HDL est calculé selon l'équation de Stokes-Einstein [3]:

$$R = \frac{K_B \cdot T}{6\pi\eta \cdot D}$$

$$[3]$$

dans laquelle

$K_B$ représente la constante de Boltzmann en J·K$^{-1}$,
T représente la température absolue en K,
η représente la viscosité de la solution en Pa·s, et
D représente le coefficient de diffusion de ladite particule de lipoprotéine HDL en cm$^2$·s$^{-1}$.

**3.** Procédé *in vitro* selon l'une quelconque des revendications 1 ou 2 comprenant une étape antérieure dans laquelle l'échantillon de liquide biologique et soumis à une spectroscopie par RMN $^1$H éditée en diffusion bidimensionnelle (2D) pour déterminer expérimentalement le rayon hydrodynamique « R » d'une particule de lipoprotéine HDL.

**4.** Procédé *in vitro* selon l'une quelconque des revendications 1 à 3, dans lequel la concentration du cholestérol libre, des phospholipides, des apolipoprotéines, des triglycérides et des esters de cholestéryle dans une particule de lipoprotéine HDL est déterminée par un procédé biochimique ou par un procédé spectroscopique.

**5.** Procédé *in vitro* selon l'une quelconque des revendications 1 à 4, comprenant en outre le calcul du pourcentage des triglycérides et des esters de cholestéryle localisés dans la coque de la particule de lipoprotéine HDL.

**6.** Procédé *in vitro* selon l'une quelconque des revendications 1 à 5, comprenant en outre le calcul du pourcentage des triglycérides et des esters de cholestéryle localisés dans le noyau de la particule de lipoprotéine HDL.

**7.** Procédé *in vitro* selon l'une quelconque des revendications 1 à 6, dans lequel l'échantillon de liquide biologique est choisi parmi le plasma sanguin, le sérum et l'une de leurs fractions.

**8.** Procédé *in vitro* selon l'une quelconque des revendications 1 à 7, dans lequel ledit procédé est effectué sur une fraction de particules de lipoprotéine HDL isolée auparavant de l'échantillon de liquide biologique.

**9.** Procédé *in vitro* selon l'une quelconque des revendications 1 à 8, dans lequel ledit procédé est effectué sur l'échantillon de liquide biologique entier.

**10.** Procédé *in vitro* pour la surveillance de la progression d'un sujet diagnostiqué avec une dyslipidémie comprenant la détermination de la distribution des triglycérides et des esters de cholestéryle entre le noyau et la coque d'une particule de lipoprotéine HDL issue d'un échantillon de liquide biologique, par un procédé selon l'une quelconque des revendications 1 à 9 et la comparaison du résultat à une mesure antérieure
dans lequel une réduction du volume de la coque d'une particule de lipoprotéine HDL occupé par les triglycérides et/ou les esters de cholestéryle par rapport aux mêmes valeurs mesurées auparavant indique que le sujet a un bon pronostic, ou
dans lequel une augmentation du volume de la coque d'une particule de lipoprotéine HDL occupé par les triglycérides et/ou les esters de cholestéryle par rapport aux mêmes valeurs mesurées auparavant indique que le sujet a un mauvais pronostic.

**11.** Procédé *in vitro* pour la surveillance de l'efficacité d'un traitement administré à un sujet diagnostiqué avec une dyslipidémie ou pour la conception d'un traitement personnalisé pour ledit sujet comprenant la détermination de la distribution des triglycérides et des esters de cholestéryle entre le noyau et la coque d'une particule de lipoprotéine HDL issue d'un échantillon de liquide biologique dudit sujet avant et après ledit traitement, par un procédé selon l'une quelconque des revendications 1 à 9

dans lequel une réduction du volume de la coque d'une particule de lipoprotéine HDL occupé par les triglycérides et/ou les esters de cholestéryle après le traitement administré par rapport aux mêmes valeurs avant le traitement indique que le traitement administré est efficace, ou

dans lequel une augmentation ou aucun changement du volume de la coque d'une particule de lipoprotéine HDL occupé par les triglycérides et/ou les esters de cholestéryle après le traitement administré par rapport aux mêmes valeurs avant le traitement indique que le traitement administré est inefficace et/ou que le sujet a besoin d'un traitement alternatif.

12. Procédé *in vitro* pour l'identification de composés appropriés pour le traitement d'une dyslipidémie comprenant la détermination dans un échantillon de liquide biologique d'un sujet souffrant d'une dyslipidémie et ayant été traité avec un composé candidat, de la distribution des triglycérides et des esters de cholestéryle entre le noyau et la coque d'une particule de lipoprotéine HDL par un procédé selon l'une quelconque des revendications 1 à 9

dans lequel une réduction du volume de la coque d'une particule de lipoprotéine HDL occupé par les triglycérides et/ou les esters de cholestéryle après le traitement avec un composé candidat par rapport aux mêmes valeurs avant le traitement avec ledit composé candidat indique que le composé est approprié pour le traitement de la dyslipidémie, ou

dans lequel une augmentation ou aucun changement du volume de la coque d'une particule de lipoprotéine HDL occupé par les triglycérides et/ou les esters de cholestéryle après le traitement avec un composé candidat par rapport aux mêmes valeurs avant le traitement avec ledit composé candidat indique que le composé n'est pas approprié pour le traitement de la dyslipidémie.

13. Procédé *in vitro* pour l'identification de composés capables de produire une dyslipidémie comprenant la détermination dans un échantillon de liquide biologique d'un sujet qui a été traité avec un composé candidat, de la distribution des triglycérides et des esters de cholestéryle entre le noyau et la coque d'une particule de lipoprotéine HDL par un procédé selon l'une quelconque des revendications 1 à 9

dans lequel une augmentation du volume de la coque d'une particule de lipoprotéine HDL occupé par les triglycérides et/ou les esters de cholestéryle après le traitement avec un composé candidat par rapport aux mêmes valeurs avant le traitement avec ledit composé candidat indique que le composé est capable de produire une dyslipidémie.

14. Procédé *in vitro* selon l'une quelconque des revendications 10 à 13, dans lequel la dyslipidémie est une dyslipidémie diabétique.

15. Programme informatique comprenant un code de programme informatique, qui, lorsqu'il est exécuté par un dispositif informatique, entraîne le dispositif informatique à effectuer les étapes du procédé selon l'une quelconque des revendications 1 à 14.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RADER, D.J. ; TALL, A.R.** *Nature Medicine,* 2012, vol. 18, 1344-1346
- **HEINECKE, J.W.** *Nature Medicine,* 2012, vol. 18, 1346-1347
- **STÄHLMAN M. et al.** Biochimica et Biophysica Acta. *Molecular and Cell Biology of Lipids,* 2013, vol. 1831 (11), 1609-1617
- **MALLOL R. et al.** *Progress in Nuclear Magnetic Resonance Spectroscopy,* 2013, vol. 70, 1-24
- **TEERLINK T.** *The Journal of Lipid Research,* 2004, vol. 45 (5), 954-966
- **KUMPULA L.S. et al.** *Chemistry and Physics of Lipids,* 2008, vol. 155 (1), 57-62
- **YETUKURI L. et al.** *The Journal of Lipid Research,* 2010, vol. 51 (8), 2341-2351
- **KUMPULA L.S. et al.** *Chemistry and Physics of Lipids,* 2008, vol. 155, 57-62
- **SKIPSKI, V.P.** Blood Lipids and Lipoproteins. Quantitation, Composition and Metabolism. Wiley-Interscience, 1972, 471-483
- **MALLOL, R. et al.** *Analytical and bioanalytical chemistry,* 2012, vol. 402, 2407-2415